# EUROPEAN PATENT APPLICATION

(11) **EP 1 580 187 A1**
(43) Date of publication of application: **28.09.2005**
(21) Application number: 04006809.0
(22) Date of filing: 22.03.2004
(51) Int. Cl.: C07C 233/18, C07D 263/06, C07D 263/52, C07D 265/32, C07D 217/24, A61P 35/00

(54) **Apoptogenic and antiproliferative analogs of ceramide**

(71) Applicant: Deigner, Hans-Peter, 68623 Lampertheim (DE)
(72) Inventor: Deigner, Hans-Peter, 68198 Schriesheim (DE); Claus, Ralf, 07747 Jena (DE); Kinscherf, Ralf, 69198 Schriesheim (DE); Riedel, Hendrik, 69123 Heidelberg (DE)
(74) Representative: Schüssler, Andrea

(57) **Abstract**

Described are analogs of ceramide which have apoptogenic and antiproliferative properties as well as pharmaceutical compositions containing these analogs. Also described is the therapeutic use of the ceramide analogs, e.g. for inhibiting proliferation, preferably in cancer therapy, or increased angiogenesis or for modulating apoptosis.

## Description

The present invention relates to novel analogs of ceramide which have apoptogenic and antiproliferative properties as well as pharmaceutical compositions containing these compounds. The present invention also relates to the therapeutic use of the ceramide analogs, e.g. for inhibiting proliferation, preferably in cancer therapy, or increased angiogenesis or for modulating apoptosis.

One class of signalling pathways that have received considerable attention in the last few years involves the generation of ceramide (Cer) by the action of cytokine- or LPS-stimulated sphingomyelinases [1-3]. Cer is an intracellular lipid mediator generated in response to a large number of extracellular signals [4-6]. These include tumor necrosis factor-alpha (TNF-α), interleukin-1β (IL-1β), ionizing and ultraviolett radiation, exposition to anti-cancer drugs, growth factor withdrawal, infection with human immunodeficiency virus (HIV) [7] or bacteria [8]. Cer is reported to participate in cell differentiation [9], senescence [10], growth arrest or programmed cell death [4-6] depending on cell type, dynamic of generation and topological restriction of involved enzymes. On the other hand, subsequent degradation of ceramide results in the formation of sphingosine, which may be further phosphorylated into sphingosine-1-phosphate. Interaction of sphingosine and sphingosine-1-phosphate with its receptors leads to an array of cellular responses including proliferation and inhibition of apoptosis. In this regard, the resulting equillibrium between ceramide, sphinogosine und sphingosine-1-phosphate (the so-called sphingolipid rheostate) has been implicated in a variety of complex and specific pathways for cellular regulation [4, 10, 11].

The role of ceramide in apoptosis has been described in lymphocytes [11] and macrophages [12], including the identification of a number of downstream targets of Cer: on posttranscriptional level, Cer-activated protein kinase (CAPK) and Cer-activated protein phosphatases (CAPP) have been identified as principal target proteins. The first one mediates effects of Cer to the transcription factors *c-myc* [13] and c-jun [14], the last one is involved in mitogen activated protein kinase cascades including extracellular-signal regulated kinases, the c-jun-N-terminal kinases, stress activated kinases (JNK, SAPK) and the family of p38 kinases [15].

At least for some cells and stresses, ceramide accumulation appears to be obligatory for apoptosis induction. Ceramide is produced by sphingomyelinases (SMases), e. g. acid SMase (aSMase); aSMase is activated by various receptors such as CD95/APO-1/Fas [16], TNF-R [17], CD5 [18] and CD28 [19]. Some cells, such as fibroblasts of Niemann-Pick patients are resistant to mmLDL-induced apoptosis [12], mouse embryonic fibroblasts from *asmase* animals, in particular *asmase*^{*-l-*} cells, proved resistance to radiation-induced apoptosis. This effect can be reverted by addition of exogenous ceramide [20]. Certain drugs inducing apoptosis in various tumor cells such as the anthracyclines daunorubicin or doxorubicin, critically involve ROS production [21] as well as ceramide accumulation [22, 23, 24]. Ceramide accumulation precedes critical steps of apoptosis such as caspase activation *e.g.* in A549 human lung adenocarcinoma cells [25] and enzymatic activities generating ceramide are involved in in acute lung failure [26]. Moreover, Cer has been reported to activate NADPH-oxidase in a direct manner contributing to Cer-induced endothelial dysfunctin in coronary arteries [27]. However, for therapy the half-life of ceramide is insufficient: ceramide is readily being metabolized by a number of enzymes, such as kinases, glucosyltransferases, sphingomyelinsynthases and ceramidases, often to compounds exhibiting partially opposite effects. Moreover, physico-chemical properties of ceramide inhibit cellular uptake and unobstructed transfer via cell membranes [9, 10].

Thus, the technical problem underlying the present invention is to provide means which mimic ceramide itself and/or increase the intracellular concentration of ceramide, exhibit anti-tumor activities and/or improve the efficiency of a treatment with additional cytostatic agents.

The solution of said technical problem is achieved by providing the embodiments characterized in the claims. During the experiments leading to the present invention novel compounds and ceramide analogs were synthesized and characterized. It was found that they can induce apoptosis and inhibit proliferation, thus, can act, e.g., as stand-alone anti-tumor agent and increase the efficiency of a cytostatic and/or radiation therapy.

The following compounds were synthesized and characterized:

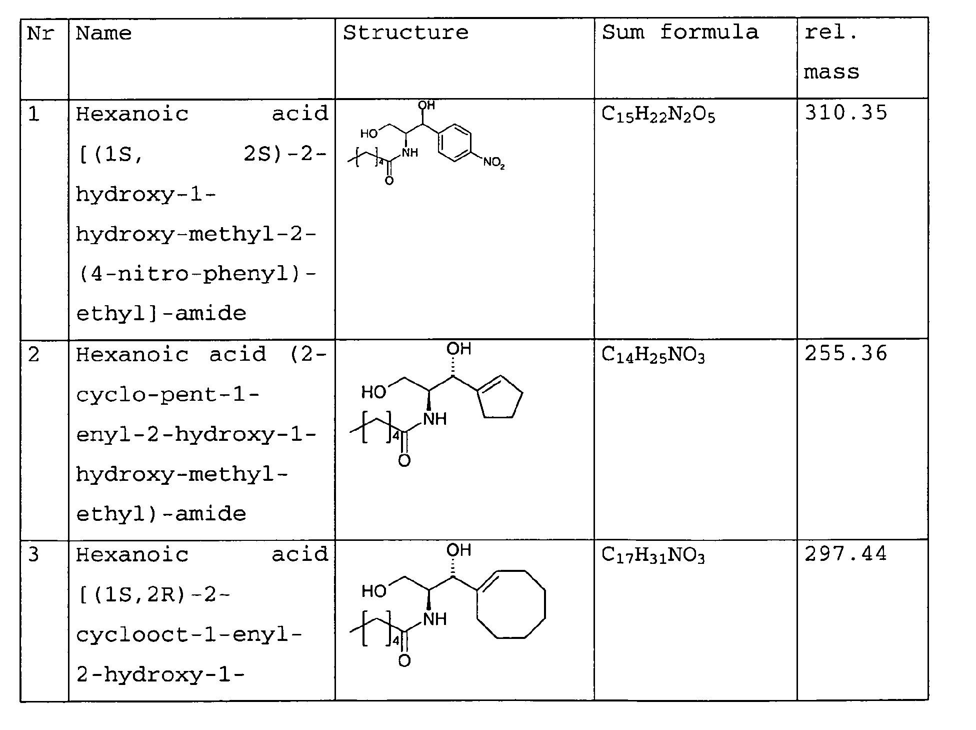

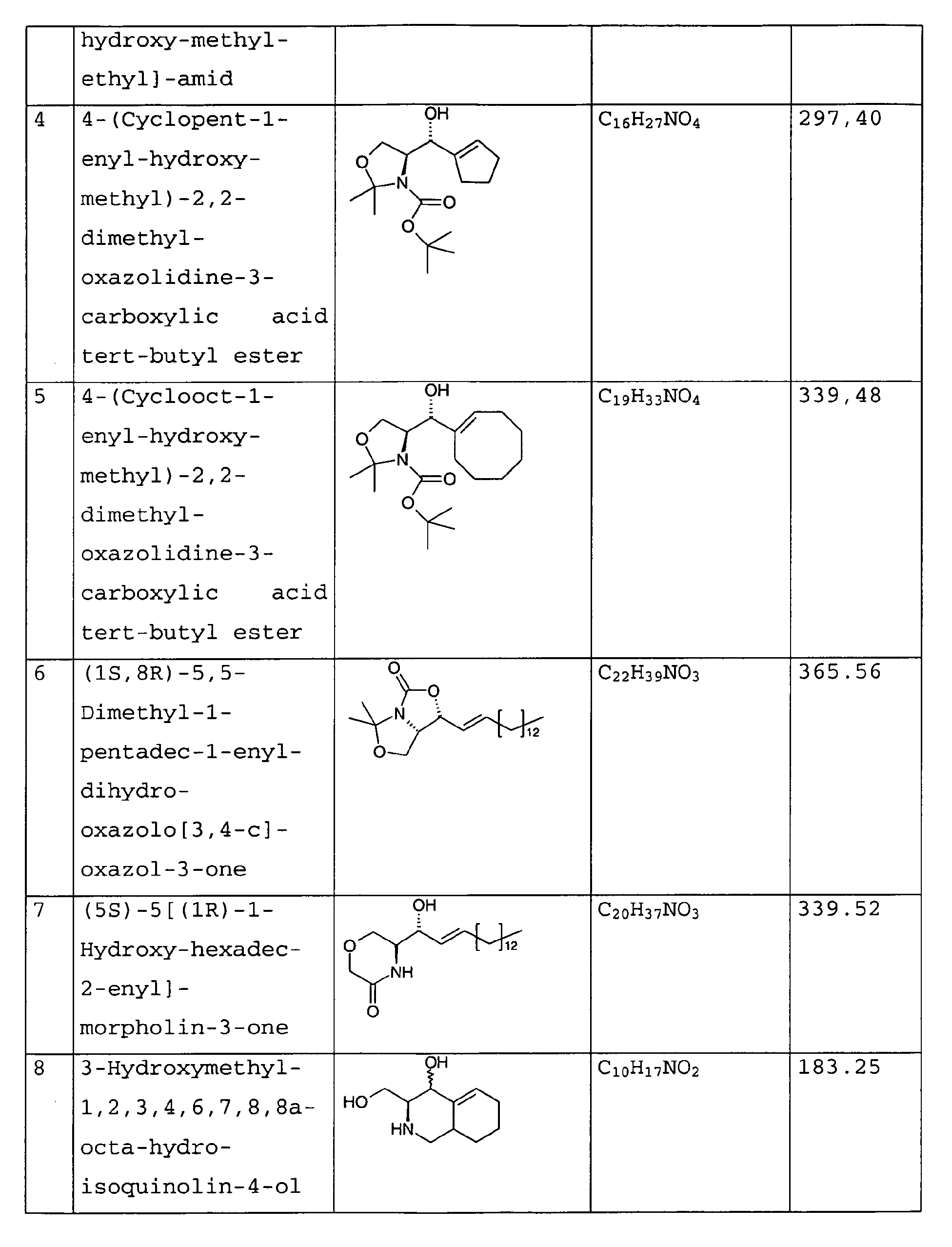

Thus, the present invention relates to a compound of the general formula A or B:

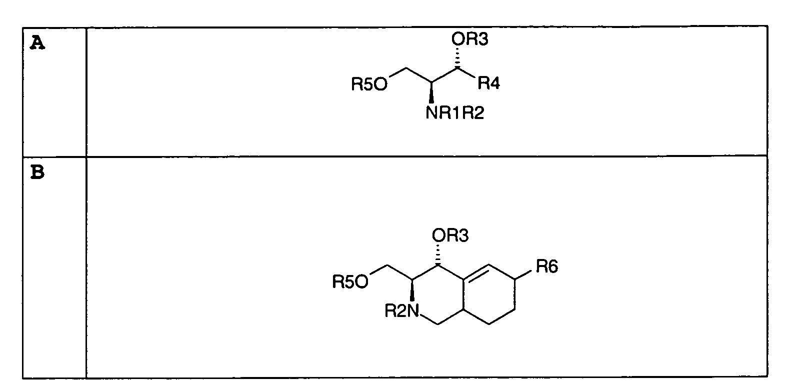

wherein
the residues R1, R2 and R3 which can be equal or different are H, CO-alkyl, CO-alkenyl, CO- aralkyl, CO-phenyl, BOC, COCF₂-alkyl, COCF₂-aralkyl, COCF₂-phenyl, a C₁-C₂₀ alkyl, alkenyl, alkinyl, aralkyl, aralkenyl residue, phenyl or substituted phenyl, a C₃-C₁₀ cycloalkyl, cycloalkenyl or substituted cycloalkenyl;
the residues R4 and R6 which can be equal or different are CO-alkyl, CO-alkenyl, CO-aralkyl, CO-phenyl, BOC, COCF₂-alkyl, COCF₂-aralkyl, COCF₂-phenyl, C₅-C₂₀ alkyl, alkenyl, alkinyl, aralkyl, aralkenyl residue, COOH, CONH₂, CONH-alkyl, CON(alkyl)₂ residue wherein alkyl - C₁-C₁₀, a nitro, an amino, alkylamino residue wherein alkyl - C₁-C₂₀, an aryl, aralkyl, alkoxyaryl residue, a halogen atom, phenyl or substituted phenyl, a C₃-C₁₀ cycloalkyl, cycloalkenyl or substituted cycloalkenyl, a C₁-C₂₀ O, S-Alkyl, - alkenyl, -alkinyl, -aralkyl, -aralkenyl ether, -phenyl or substituted phenyl ether, a C₃-C₈ O, S-cycloalkenyl or substituted cycloalkenylether, -epoxyalkyl, -aryl, -alkenyl, or aziridinoalkyl, -aryl, -alkenyl; and
the residue R5 is an substituted or unsubstituted alkyl, alkenyl, alkinyl, aralkyl, aralkenyl phosphate ester, phosphonic- or phosphinic acid ester, a sulfonic- or sulfinic-acid ester or a biologically active isostere thereof;
or a pharmaceutically acceptable salt thereof.

In the present invention, the term "alkyl residue" relates to an unbranched, branched or (poly)cyclic alkyl chain, preferably C₁ - C₂₀, comprising e.g. the methyl, ethyl, n-propyl, isopropyl, n-butyl, isobutyl, sec-butyl, tert-butyl, n-pentyl, isopentyl, neopentyl, tert-pentyl, 2-methylbutyl, n-hexyl, isohexyl, 2-methylpentyl, 2,3-dimethylbutyl, n-heptyl, 2-methylhexyl, 2,2-dimethylpentyl, 3,3-dimethylpentyl, 3-ethylpentyl, n-octyl, 2,2-dimethylhexyl, 3,3-dimethylhexyl, 3-methyl-3-ethylpentyl, cyclopropyl, cyclobutyl, cyklopentyl, cylohexyl, cycloheptyl, cyclooctyl group. Short alkyl chains, such as methyl, ethyl or propyl are preferred. The same applies correspondingly to the o-alkyl residue.

In the present invention, the term "alkenyl residue" relates to an unbranched, branched or (poly)cyclic alkenyl chain, preferably C₂ - C₂₀, comprising, such as, for example, vinyl, propenyl, isopropenyl, allyl, 2-methylallyl, butenyl, isobutenyl, pentenyl or hexenyl, isohexenyl, heptenyl, isoheptenyl, octenyl, isooctenyl groups, cyclobutenyl, cyclopentenyl, cyclohexenyl, cycloheptenyl, cyclooctenyl, cyclononenyl or cyclodecyl groups. Vinyl, propenyl and isopropenyl are preferred.

In the present invention, the term "alkinyl residue" relates to an unbranched, branched or (poly)cyclic alkinyl chain, preferably C₂ - C₂₀, comprising, such as, for example, ethinyl, propinyl, isopropinyl, butinyl, isobutinyl, pentinyl, isopentinyl or hexinyl groups.

Polycyclic alkyl or alkenyl residues, respectively, comprise norbornane, adamantane or benvalene.

In the present invention, the term "aryl residue" refers to a mono- or polycyclic C₆ - C₂₀ aryl residue. Examples thereof are a carbocyclic, monocyclic residue, e.g. the phenyl group, a heterocyclic, monocyclic residue, e.g. the groups thienyl, furyl, pyranyl, pyrrolyl, imidazolyl, pyrazolyl, pyridyl or pyrazinyl. These aryl residues may be unsubstituted or substituted. Suitable substitutents are C₁ - C₄ alkyl groups (as defined above), halogen, nitro, hydroxy, carboxy, amino, carbamoyl, C₂ - C₆ alkenyl or alkinyl groups (as defined above).

The term "aralkyl residue" comprises, for example, the phenylethyl, phenylpropyl phenylbutyl or phenylpentyl group.

In the present invention, the term "alkoxy residue" refers, for example, to a methoxy, ethoxy, propyloxy, isopropyloxy or butyloxy group.

In the present invention, the term "halogen" comprises fluorine, chlorine, bromine and iodine, whereas chlorine and bromine are preferred.

The term "pharmaceutically acceptable salt" comprises the salts of inorganic acids, such as, for example, hydrochloride, hydrobromide, sulfate, perchlorate, nitrate and phosphate, as well as the salts of organic acids, such as, for example, acetate, oxalate, succinate, tartrate, maleate and fumarate.

The compounds according to the invention can be produced by the methods of Example 2 (final compounds 1 to 8) described below. Their therapeutic suitability, e.g. for the improvement of the effect of cytostatic and/or radiation therapy and the optimized concentration can be verified by the methods described in Example 1 below (inhibition of proliferation and induction of apoptosis by different methods).

Preferred compound are compounds in which the structures contain alkyl-, alkenyl-bridges as indicated in the general formula B and/or unsubstituted or substituted alkyl-, alkylen-bridges between the heteroatoms as shown, for example, in compounds 4, 6 and 7.

Particularly preferred are the following compounds:
(1) Hexanoic acid [(1S,2S)-2-hydroxy-1-hydroxy-methyl-2-(4-nitro-phenyl)-ethyl]-amide 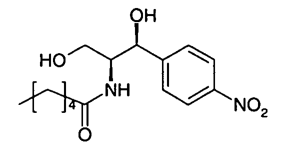
(2) Hexanoic acid (2-cyclo-pent-1-enyl-2-hydroxy-1-hydroxymethyl-ethyl)-amide 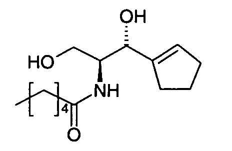
(3) Hexanoic acid [(1S,2R)-2-cyclooct-1-enyl-2-hydroxy-1-hydroxy-methyl-ethyl]-amid 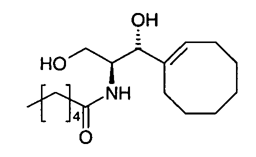
(4) 4-(Cyclopent-1-enyl-hydroxy-methyl)-2,2-dimethyloxazolidine-3-carboxylic acid tert-butyl ester 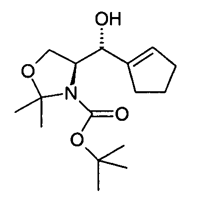
(5) 4-(Cyclooct-1-enyl-hydroxy-methyl)-2,2-dimethylboxazolidine-3-carboxylic acid tert-butyl ester 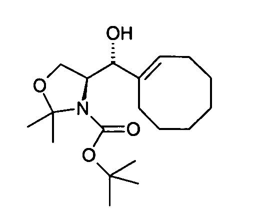
(6) (1S,8R)-5,5-Dimethyl-1-pentadec-1-enyl-dihydrooxazolo[3,4-c]-oxazol-3-one 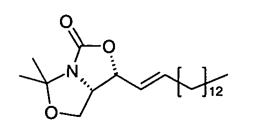
(7) (5S)-5[(1R)-1-Hydroxy-hexadec-2-enyl]-morpholin-3-one 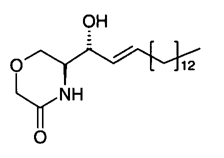 or
(8) 3-Hydroxymethyl-1,2,3,4,6,7,8,8a-octa-hydro-isoquinolin-4-ol

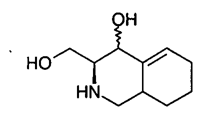

The present invention also relates to a pharmaceutical preparation which contains a pharmacologically active amount of a compound according to the invention together with a pharmaceutically acceptable carrier. Suitable carriers and the formulation of such pharmaceutical preparations are known to a person skilled in the art. Suitable carriers comprise e.g. phosphate-buffered common salt solutions, water, emulsions, e.g. oil/water emulsions, wetting agents, sterile solutions, etc. The pharmaceutical preparation according to the invention can be available in the form of an injection solution, tablet, ointment, suspension, emulsion, a suppository, etc. It can also be administered in the form of depots (microcapsules, zinc salts, liposomes, etc.). The kind of administration of the pharmaceutical preparation depends *inter alia* on the form in which the active substance is available; it may be oral or parenteral. The methods of parenteral administration comprise the topical, intra-arterial, intramuscular, intramedullary, intrathekal, intraventricular, intravenous, intraperitoneal, transdermal or transmucosal (nasal, vaginal, rectal, and sublingual) administration. The administration can also be made by microinjection. The suitable dose is determined by the attending physician and depends on various factors, e.g. on the patient's age, sex and weight, the kind and stage of the disease, e.g. the tumor, the kind of administration, etc.

Finally, the present invention also relates various therapeutic uses of a compound according to the invention.

They can be used for stand alone cancer therapy, and or improving the efficiency of cytostatic and/or radiation therapy or for the prevention or reduction of resistance formation in a cytostatic and/or radiation therapy in which, preferably, the tumor cells are pre-treated previous to the start of the therapy with a compound according to the invention.

The compounds according to the invention are in principle suitable for the combination with a radiation therapy. Further, they can be applied in a cytostatic therapy with each cytostatic agent in which cytostatic agents are preferred for which effect the formation of ceramide or the increase of the concentration of ceramide in the tumor cells is required. Examples for suitable cytostatic agents are vinblastine, vincristine, vinorelbine, vindesine and other vinca-alkaloides or analogs thereof, respectively, daunorubicin, doxorubicin, aclarubicin, epirubicin and other anthracyklines or analogs thereof, respectively, whereas daunorubicin or vinblastine are preferred.

Thus, the present invention relates to
(a) Use of a compound according to the invention for the preparation of a pharmaceutical composition for cancer therapy and/or for improving the efficiency of a cytostatic and/or radiation therapy.
(b) Use of a compound according to the invention for the preparation of a pharmaceutical composition for preventing or reducing the formation of resistances by a cytostatic and/or radiation therapy. A preferred cytostatic agent is an intercalating agent or antimitotic agent. A preferred intercalating agent is daunorubicin and a preferred antimitotic agent is a vinca alcaloid, preferably vinblastine.
(c) Use of a compound according to the invnetion for the preparation of a pharmaceutical composition for inhibiting proliferation of and/or inducing apoptosis in mammalian, preferably human cells.
(d) Use of a compound according to the invention for the preparation of a pharmaceutical composition for therapy and/or prophylaxis of cancer (e.g., a lung adenocarcinoma), colorectal hyperproliferation or inhibition of increased angiogenesis. In a preferred embodiment, said cancer therapy is an adjuvant therapy wherein said compound is combined with at least one additional anti-cancer agent of an independent or complementary mechanism.
(e) Use of a compound according to the invention for the preparation of a pharmaceutical composition for therapy and/or prophylaxis of asthma, rheumatoide arthritis, autoimmune arthritis, morbus crohn, colitis ulcerosa, systemic inflammation, sepsis, acute respiratory distress syndrome (ARDS), chronic obstructive and/or restrictive lung disease or a disease associated with hyperproliferation and/or epithelial disorder.
   Preferably, said epithelial disorder is psoriasis, comedogenesis, chronic proliferative dermatitis, epithelializating or chronic wounds, systemic lupus erythematosus, endometriosis or chronic irritant contact dermatitis.
(g) Use of a compound according to the invention for the preparation of a pharmaceutical composition for inhibiting proliferation of vascular cells in vessels treated with stents impregnated with said compounds and/or containing devices for retarded or continous delivery.
(h) Use of a compound according to the invention for the preparation of a pharmaceutical composition for inducing positive inotropic effects in ventricular myocytes or for an improvement of the left ventricular function.
(i) Use of a compound according to the invention for the preparation of a pharmaceutical composition for (a) preventing or attenuating apoptosis of neuronal cells or (b) inhibiting or reducing the consequences of stroke and/or ischaemic insult.
(j) Use of a compound according to the invention for the preparation of a pharmaceutical composition for therapy, adjuvant therapy and prophylaxis of neurodegenerative disorders such as morbus alzheimer, m. parkinson, a prion disease or amyotrophic lateral sclerosis.

Finally, the present invention also relates to the use of a compound according to the present invention for the preparation of a pharmaceutical composition for prophylaxis, therapeutically monitoring and its use as an adjuvant therapeutic in cancer therapy, colorectal hyperproliferation, asthma, rheumatoide arthritis, autoimmune arthritis, Morbus Crohn, colitis ulcerosa, systemic inflammation, sepsis, acute respiratory distress syndrome (ARDS), chronic obstructive and/or restrictive lung disease, a disease associated with hyperproliferation and/or epithelial disorder.

### Example 1: General Methods

### (A) Cell culture

Human peripheral blood mononuclear cells from venous blood of healthy volunteers were prepared by Ficoll-Paque density gradient centrifugation as described by the manufacturer's instructions and cultivated in RPMI 1640 medium supplemented with FCS (10%), glutamine and penicillin/streptomycin for 5 days. Non-adherent cells were removed by washing with supplemented RPMI medium. Differentiated MΦ were transferred to human AB-serum (1%) for 16 h and then exposed to the respective stimuli; detailed conditions are given in the legends to the Figures.

Human colon carcinoma cells HT-29 were purchased from ATCC and cultivated under standard conditions (37°C, 5% CO₂, McCoy 5A medium, 10% fetal calf serum, penicillin, streptomycin). For experimentation, cells were seeded in cell culture dishes at appropriate density, stimulated and analysed as described in the following section.

### (B) Proliferation

Quantitative determination of DNA synthesis in replicating cells was analyzed using commercial available test kits (colorimetric BrdU-ELISA, Roche-Diagnostics) according to the manufacturer's instructions. For these experiments, HT-29 colon carcinoma cells were seeded in 96-well plates at 40% confluence, cultivated overnight (1% FCS), incubated for 16 h with the test compounds (concentration range: three orders of magnitude), pulsed labelled with BrdU and fixed. The amount of incorporated BrdU was analysed using specific antibodies (Roche Diagnostics, Mannheim, Germany) and subsequent colorimetric reaction. The rate of DNA-synthesis in untreated control cells with standard medium was determined and compared with that in a medium contained the test compounds.

### (C) Apoptosis

Apoptotic macrophages and colon carcinoma cells were identified by microscopic observation of typical morphological changes (blebbing, generation of apoptotic bodies) and quantitated by YOPRO-1 staining [28] and Hoechst 33342 dye. The percentage of apoptotic cells was counted using a microscope fitted with mercury light source and fluorescence assembly in combination with a computer-assisted morphometry system [12]. Apoptosis was confirmed by PARP cleavage detected by Western Blotting [29] and single cell electrophoresis (COMET-assay) [30].

### (D) Western Blotting

Cell extracts were prepared from 2 x 10⁶ stimulated cells (4 h, 20 µM). Cells were washed once with ice-cold PBS, lysed in sample buffer (62.5 mM TRIS-HCl, pH 6.8, 6M urea, 10% Glycerol, 2% SDS, 0.00125% bromophenolblue, 5% mercaptoethanol), sonicated for 15 sec and incubated at 65°C for 15 min. After running a SDS-PAGE (2 µl lysate on a 10% gel), proteins were blotted to PVDF membrane (16 h, 500 mAh) and blocked with 1% BSA in TBST (50 mM TRIS-HCl, pH 7.4; 150 mM NaCl; 0.1% Tween 20) with gentle agitation. After incubation with anti-PARP-antibody (BIOMOL Research Laboratories, Hamburg, Germany; 1:200 in TBST, 1 % BSA) for 12 h, proteins were visualised after extensive washing with TBST and incubation with HRP conjugated anti-mouse secondary antibody using the ECLplus system from Amersham (Freiburg, Germany).

### (E) COMET assay

After incubation of PBMC or HT-29 with the stimuli as indicated in the Figure legend (final volume 25 *µ*l), alkaline lysis and denaturation, slides were placed on a horizontal electrophoresis unit. Electrophoresis was conducted for 20 min (25 V, 300 mA). After neutralisation and staining with ethidium bromide, DNA migration was analyzed on a Olympus Provis microscope with fluorescence equipment and measure dwith a scaled ocular. For evaluation of migration, 60 cells were scored [30].

### (F) SMase activity determination

Activities of aSMase and nSMase were determined by hydrolysis of a fluorogenic C₁₂-analogue substrate [(7-Nitro-2-1,3-benzoxadiazol-4-yl)amino-dodecyl-D-erythro-sphingosine, NBD-sphingomyelin, Molecular Probes, subsequent separation of the labelled NBD ceramide analog by thin layer chromatography and data analysis at conditions as described in detail previously [31].

### (G) Statistical analysis

Statistical analyses performed include the Student t tests and Mann Whitney U tests.

### Example 2: Synthesis and characterization of ceramid analogs

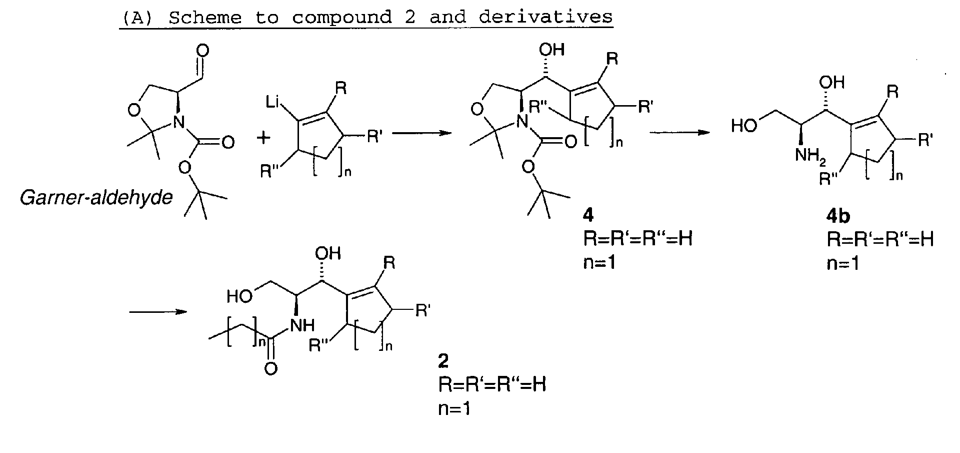

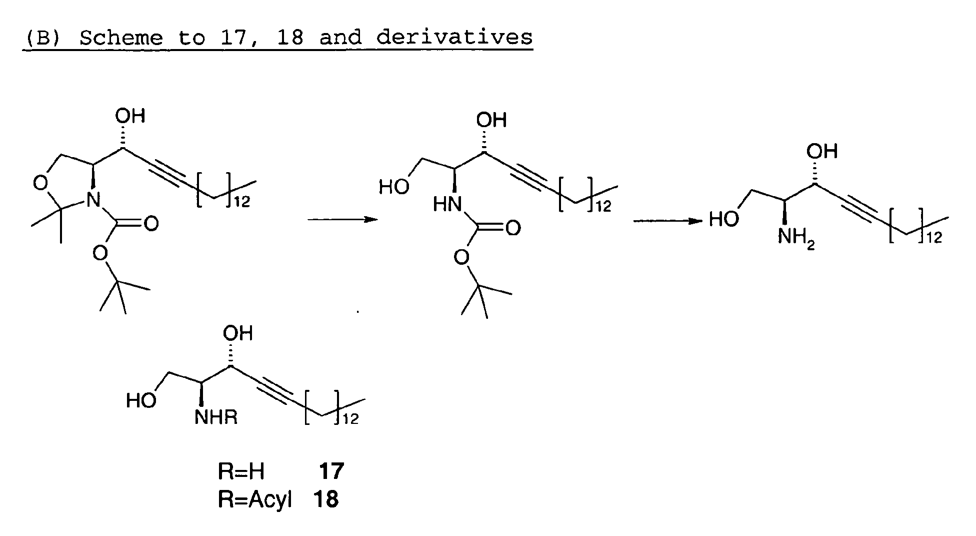

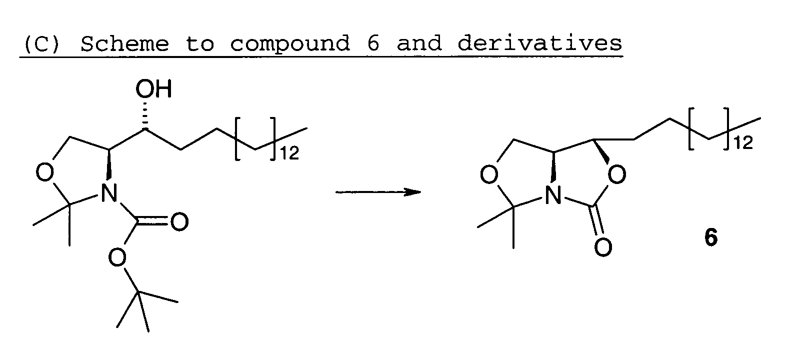

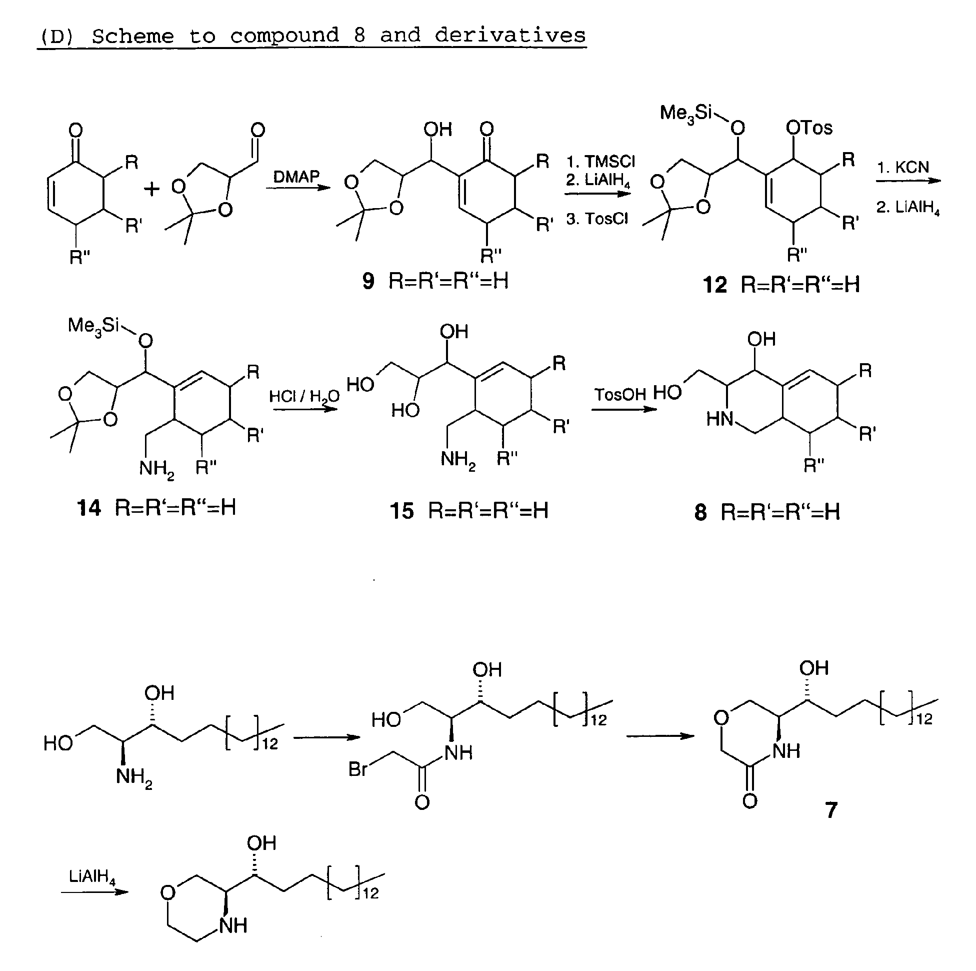

### (D) Synthesis

### Hexanoic acid [(1S,2S)-2-hydroxy-1-hydroxymethyl-2-(4-nitrophenyl)-ethyl]-amide (1)

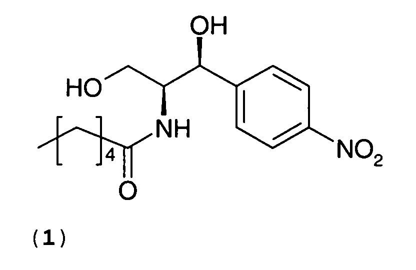

2.00 g (9.42 mmol) (2S,3S)-2-Amino-3-(4-nitrophenyl)-propane-1,3-diol are solved in 200 ml THF, 10.0 ml (72.3 mmol) triethylamine added and the solution cooled to -78 °C. 1.27 g (9.42-mmol) caproylchloride in 50 ml THF are slowly added and the solution stirred for 30 min at the same temperature. The reaction is quenched with 250 ml of saturated NaHCO₃ solution. The mixture is extracted three times with 250 ml Ethylacetate. The combined organic layers are washed with 300 ml water and dried over MgSO4. Evaporation of the solvent and chromatographic purification (silica gel // CH₂Cl₂:methanol = 9:1, R_{f}(1) = 0.51) gives 2.80 g (9.02 mmol, 95%) pure 1 as a slightly yellowish oil.

^{**1**}**H-NMR** (360 MHz, CDCl₃, 297 K): δ [ppm] = 0.79 (t, 3H, ³*J* = 7.0 Hz), 1.00-1.46 (m, 6H),2.07 (t, 2H, ^{*3*}*J= 3.9* Hz), 3.76 (m, 2H), 4.16 (m, 1H), 4.26 (br, 2H), 5.18 (d, 1H, ^{*3*}*J =* 2.7 Hz), 6.40 (d, 1H, ³*J* = 8.7 Hz), 7.54 (d, 2H, ^{*3*}*J =* 8.7 Hz), 8.13 (d, 2H, ³*J* = 8.7 **Hz);**^{**13**}**C-NMR** (90.56 MHz, CDCl₃, 297 K): δ [ppm] = 13.7, 22.2, 25.2, 31.0, 36.5, 55.9, 62.7, 71.7, 123.6, 126.7, 147.1, 149.2, 174.5; **MS** (EI): m/z (%) = 311 (0.17) [MH⁺], 310 (0.01) [M⁺], 293 (0.59), 261 (0.68), 236 (0.75), 181 (5), 158 (16), 99 (14), 71 (17), 60 (100), 55 (10), 43 (55); **IR** (film): 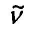 = 3339 (br), 2959 (s), 2931 (s), 2872 (w), 1750 (w), 1651 (s), 1520 (s), 1467 (m), 1348 (s), 1259 (m), 1078 (s), 1048 (s), 850 (m), 709 (m) cm⁻¹; **HRMS** (CI): calculated for C₂₃H₄₃N₂O₅ [M+H⁺] : 311.1607, found: 311.1605 (-0.6 ppm).

### (S)-4-((R)-Cyclopent-1-enyl-hydroxy-methyl)-2,2-dimethyloxazolidine-3-carboxylic acid tert-butyl ester (4)

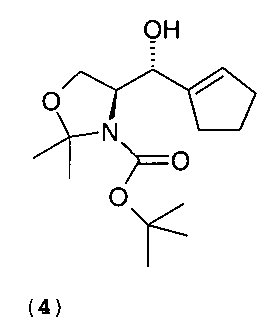

15.8 ml (1.5 M, 23.7 mmol) of tert-Butyllithium-solution in tetrahydrofurane are added dropwise to a stirred solution of 1.74 g (11.8 mmol) 1-bromcyclopentene in 20 ml absolute THF at -78 °C . After 2 h, 678 mg (2.96 mmol) Garner aldehyde in 10 ml THF are added while the temperature is kept constant. The solution is allowed to warm to RT over night and quenched by the addition of 10 ml saturated ammoniumchloride. The mixture is extracted with ethylacetate (3 x 50 ml), the organic layer is separated and the combined organic phases washed with 50 ml brine and dried over magnesiumsulfate. After filtration, the solvent is evaporated and column chromatography on silca (n-hexane:ethylacetate = 4:1, **R**_{**f**}**(4) =** 0.32) affords 687 mg (2.31 mmol, 78 %) of colourless oil **4.**

^{**1**}**H-NMR** (360 MHz, CDCl₃, 297 K): δ [ppm] = 1 .40-1 . 60~ (m, 2H), 1.48 (s, 9H), 1.50 (s, 6H), 2.13 (quin, 2H, ³*J* = 7.1 Hz), 2.24 - 2.37 (m, 2H), 3.70 - 3.76 (m, 1H), 4.12 (q, 1H, ³*J* = 7.2 Hz, ²*J* = 7.2 Hz), 4.52-4.60 (m, 1H), 5.62-5.68 (m, 1H); ¹³ **C-NMR** (90.56 MHz, CDCl₃, 297 K): δ [ppm] = 23.1, 24.4, 28.4, 32.1, 32.2, 60.8, 65.2, 71.8, 86.2, 94.3, 129.1, 144.0, 161.7; **MS** (CI+, i-Butan): m/z = 298 [MH⁺], 280, 264, 240, 200, 198, 184, 100; **IR** (film): 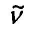 = 3483 (br), 3059 (s), 2881 (s), 1705 (s), 1620 (s), 1468 (s), 1386 (s), 1263 (m), 1129 (s), 1046 (s), 952 (s), 835 (s), 791 (s) cm⁻¹; **HRMS** (CI): calculated for C₁₆H₂₈NO₄ [M+H⁺] : 298.2018, found: 298.2016 (-0.7 ppm).

### ((1S,2R)-2-Cyclopent-1-enyl-2-hydroxy-1-hydroxymethyl-ethyl)-carbamic acid tert-butyl ester (4a)

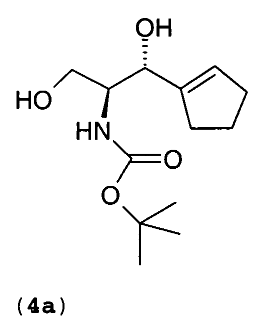

320 mg (1.08 mmol) of compound 4 are solved in 25 ml of methanol and 2.40 g Amberlyst 15 are added. The reaction mixture is strirred for 48 h, filtered over Celite and evaporated. The crude product is purified by column chromatography (silica gel // *n*-Hexane:Ethylacetate = 2:1, R_{f}(**4a**) = 0.45). The yield of product **4a** is 71 % (203 mg, 788 *µ*mol).

^{**1**}**H-NMR** (360 MHz, CDCl₃ 297 K): δ [ppm] = 1.45-1.54 (m, 2H), 1.48 (s, 9H), 2.16 (quin, 2H, ³*J* = 7.2 Hz), 2.23-2.36 (m, 2H), 3.69-3.73 (m, 1H), 4.17 (dd, 1H, ³*J* = 7.3 Hz, ²*J* = 14.5 Hz), 4.51-4.62 (m, 1H), 5.60-5.69 (m, 1H); ^{**13**}**C-NMR** (90.56 MHz, CDCl₃, 297 K): δ [ppm] = 24.5, 28.4, 32.1, 32.4,59.4, 62.5, 79.2, 79.9, 129.7, 140.0, 155.1; **MS** (CI+, *i*-Butane): m/z = 258~[MH⁺], 240, 224, 200, 198, 184, 100; **IR** (film): 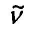 = 3475 (br), 3054 (s), 2879 (s), 1704 (s), 1626 (s), 1468 (s), 1268 (m), 1126 (s), 1016 (s), 976 (s), 785 (s) cm⁻¹; HRMS (CI): calculated for C₁₃H₂₄NO₄ [M+H⁺] : 258.1705, found: 258.1706 (+0.4 ppm).

### (1R,2S)-2-Amino-1-cyclopent-1-enyl-propane-1,3-diol (4b)

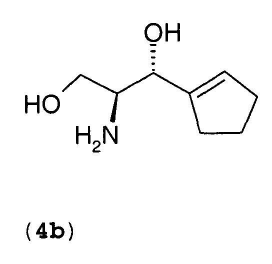

A solution of 150 mg (583 µmol) **4a** in 10 ml 1,4-dioxan is added to 5.0 ml 1N HCl and heated to 100 °C for 40 min. After cooling to room temperature the solvent is evaporated followed by chromatographically workup (silica gel // CH₂Cl₂:methanol = 3:1, **R**_{**f**}**(4b) =** 0.62) resulting in 64.2 mg (408 µmol, 70 %) pure product 4b as a colourless oil.

^{**1**}**H-NMR** (360 MHz, d₆-DMSO, 297 K): δ [ppm] = 1.40-1.60 (m, 2H), 2.23 (quin, 2H, ^{*3*}*J =* 7.1 Hz), 2.38-2.47 (m, 2H), 3.15-3.26 (m, 1H), 3.32-3.41 (m, 1H), 3.75 (m, 2H), 5.67-5.81 (m, 1H); ¹³**C-NMR** (90.56 MHz, d$_6$-DMSO, 297 K): δ [ppm] = 24.6, 32.1, 34.6, 56.9, 64.5, 75.1, 126.0, 136.3; **MS** (CI+, i-Butane): m/z = 158 [MH⁺], 140, 122, 100; **IR** (film): 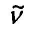 = 3413 (br), 3048 (s), 2885 (s), 1626 (s), 1469 (s), 1086 (s), 956 (s), 833 (s), 790 (s) cm⁻¹; **HRMS** (CI) : calculated for C₈H₁₆NO₂ [M+H⁺] : 158.1181, found: 158.1183 +1.3 ppm.

### Hexanoic acid ((1S,2R)-2-cyclopent-1-enyl-2-hydroxy-1-hydroxymethyl-ethyl)-amide (2)

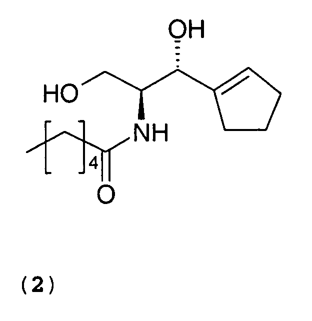

0.50 ml (3.61 mmol) triethylamine is added to a solution of 40 mg (254 *µ*mol) (1R,2S)-2-Amino-1-cyclopent-1-enyl-propane-1,3-diol (**4b**) in 25 ml THF. The resulting mixture is cooled to -78 °C. 34.2 mg (254 *µ*mol) caproylchloride in 5 ml THF are slowly dropwise added and the solution stirred for 40 min at -78 °C. The reaction is quenched with 25 ml of a saturated NaHCO₃ solution. The mixture is extracted three times with 100 ml CH₂Cl₂, the combined organic layers dried over MgSO₄ and the solvent evaporated. The crude product is purified by column chromatography (silica gel // n-hexane:ethylacetate = 4:1, R_{f}(**2**) = 0.38). The amount of isolated pure product 2 is 61.0 mg (239 *µ*mol, 94 %).

^{**1**}**H-NMR** (360 MHz, CDCl₃, 297 K): δ [ppm] = 0.88 (t, 3H, ³*J* = 7.0 Hz), 1.29-1.80 (m, 8H), 2.18-2.37 (m, 6H), 3.51 (dd, 1H, ³*J* = 3.8 Hz, ³*J* = 11.1 Hz), 3.91 (dt, 1H, ³*J* = 3.8 Hz, ³*J* = 7.4 Hz), 3.97 (dd, 1H, ³*J* = 3.7 Hz, ^{*2*}*J* = 11.1 Hz), 4.02 (d, 1H, ³*J* = 7.3 Hz), 4.12 (q, 1H, ³*J* = 7.3 Hz, ²*J* = 7.3 Hz), 5.59-5.69 (m, 1H), 6.28 (d, 1H, ³*J* = 7.5 Hz); ^{**13**}**C-NMR** (90.56 MHz, CDCl₃, 297 K): δ [ppm] = 14.1, 22.5, 26.7, 27.2, 31.5, 32.0, 33.7, 36.1, 60.3, 62.9, 77.3, 128.5, 136.9, 174.2; MS (CI+, *i*-Butane): m/z = 256 [MH⁺], 238, 195, 98, 69; **IR** (KBr): 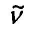 = 3438 (br), 2928 (s), 2876 (s), 1708 (s), 1664 (s), 1572 (s), 1386 (m), 1261 (m), 1090 (s) cm⁻¹; **HRMS** (CI): calculated for C₁₄H₂₆NO₃ [M+H⁺] : 256.1913, found: 256.1912 (-0.4 ppm).

### (S)-4-[(R)-((E)-Cyclooct-1-enyl)-hydroxy-methyl]-2,2-dimethyloxazolidine-3-carboxylic acid tert-butyl ester (5)

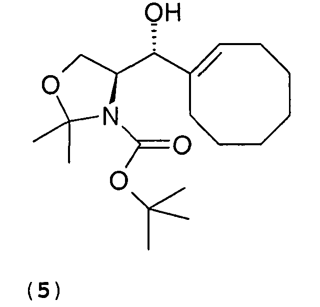

21.4 ml (1.5 M, 32 mmol) of a tert-butyllithium solution in pentane are slowly added dropwise to a solution of 3.01 g (16 mmol) of 1-bromo-cylcooctene in 20 ml THF. After 2 h at the same temperature 917 mg (4.00 mmol) *Garner aldehyde* in 10 ml THF are added. The mixture is allowed to warm to room temperature overnight and the reaction quenched afterwards by addition of 10 ml of a saturated NH₄Cl solution. The organic layer is separated and the aqueous phase extracted with brine three times. The combined organic layers are dried over MgSO₄, filtered and the solvent evaporated. Column chromatography on silca gel (n-hexane:ethylacetate = 4:1, R_{f}(**5**) = 0.55) yields 855 mg (2.52 mmol, 63 %) of a solid colourless product (5).

^{**1**}**H-NMR** (360 MHz, CDCl₃, 297 K): δ [ppm] - 1.30-1.45 (m, 2H), 1.45-1.61 ~ (m, 4H), 1.51 (s, 9H), 1.61 (s, 6H), 1.65-1.78 (m, 2H), 2.13 (dt, 2H, ³*J* = 7.9 Hz, ³*J* = 4.6 Hz), 2.19-2.32 (m, 2H), 3.73-3.88 (m, 1H), 4.08 (d, 1H, ³*J* = 4.1 Hz), 4.71 (m, 1H), 5.63 (t, 1H, ³*J* = 8.4 Hz); ^{**13**}**C-NMR** (90.56 MHz, CDCl₃, 297 K): δ [ppm] = 24.2, 24.9, 25.9, 26.4, 27.5, 28.4, 28.9, 30.5, 60.6, 65.4, 81.7, 82.6, 94.4, 130.0, 140.7, 156.0; **MS** (CI+, *i*-Butane): m/z = 340 [MH⁺], 322, 284, 282, 266, 240, 226, 200, 144, 100 ; **IR** (KBr): 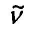 = 3460 (br), 2926 (s), 2853 (s), 1701 (s), 1459 (m), 1362 (s), 1246 (m), 1115 (s), 848 (m), 769 (m) cm⁻¹; **HRMS** (CI): calculated for C₁₉H₃₄NO₄ [M+H⁺] : 340.2488, found: 340.2487 (-0.3 ppm).

### [(1S,2R)-2-((E)-Cyclooct-1-enyl)-2-hydroxy-1-hydroxymethyl-ethyl]-carbamic acid tert-butyl ester (5a)

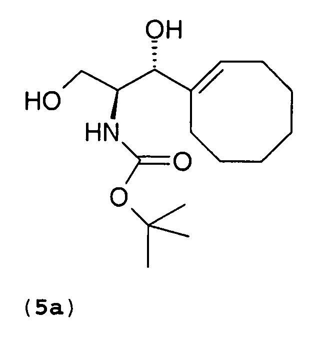

400 mg (1.18 mmol) 5 are solved in 25 ml methanol and 2.50 g Amberlyst 15 are added. The reaction mixture is stirred for 48 h at room temperature, the catalyst removed by filtration over Celite and the filtrate concentrated to dryness. The crude product is filtrated over silica gel (*n-hexane:ethylacetate =* 1:1) and recristallized form n-hexane/ ethylacetate. 238 mg (813 *µ*mol, 69 %) of pure solid **5a** are obtained.

^{**1**}**H-NMR** (360 MHz, d₆-DMSO, 297 K): δ [ppm] = 1.28-1.46 (m, 2H), 1.64-1.79 (m, 2H), 2.05 (dt, 2H, ³*J* = 7.9 Hz, ³*J* = 4.6 Hz), 2.21-2.30 (m, 2H), 3.25-3.38 (m, 1H), 3.40-3.56), 3.83 (q (br), 1H, ³*J* = 6.7 Hz), 4.39 (t, 1H, ³*J* = 5.7 Hz), 4.76 (d, 1H, ³*J* = 4.7 Hz), 5.73 (t, 1H, ^{*3*}*J* = 7.9 Hz), 6.18 (d, 1H, ³*J* = 9.0~Hz); ^{**13**}**C-NMR** (90.56 MHz, d₆-DMSO, 297 K): δ [ppm] = 25.2, 26.1, 26.5, 28.7, 28.9, 29.6, 58.8, 62.5, 79.6, 80.3, 132.1, 141.9, 155.6; MS (CI+, *i-Butan):* m/z = 300 [MH⁺], 282, 268, 244, 226, 182, 165, 143; **IR** (KBr): 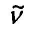 = 3459 (s), 3272 (s), 2953 (s), 2928 (s), 2851 (s), 1703 (s), 1466 (m), 1409 (m), 1262 (m), 1119 (m), 1090 (m), 1012 (m), 964 (m), 773 (m) cm⁻¹; **HRMS** (CI): calculated for C₁₆H₃₀NO₄ [M+H⁺] : 300.2175, found: 300.2174 (-0.3 ppm).

### (1R,2S)-2-Amino-1-((E)-cyclooct-1-enyl)-propane-1,3-diol (5b)

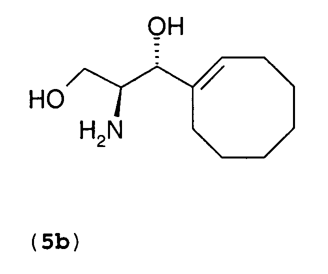

The synthesis of substance 5b is carried out simular to the procedure for substance 4b. The yield is 68 %.

^{**1**}**H-NMR** (360 MHz, CDCl₃, 297 K) : δ [ppm] = 1.31-1.59 (m, 6H), 1.63-1.76 (m, 2H), 2.11 (dt, 2H, ³*J* = 7.6 Hz, ³*J* = 4.9 Hz), 2.19-2.32 (m, 2H), 2.49 (br, 4H), 2.87 (quin., 1H, ³*J* = 5.5 Hz), 3.66 (m, 2H), 3.84 (d, 1H, ³*J* = 6.4 Hz), 5.65 (t, 1H, ³*J* = 8.6 Hz); ^{**13**}**C-NMR** (90.56 MHz, CDCl₃, 297 K): δ [ppm] = 25.3, 25.6, 26.8, 29.0, 30.2, 56.2, 64.0, 76.3, 124.1, 132.3; **MS** (CI+, i-Butan): m/z = 200 [MH⁺], 182, 174, 165, 148, 139, 113, 87; **IR** (KBr): 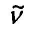 = 3376 (br), 3248 (br), 2924 (s), 2852 (s), 1578 (s), 1465 (m), 1098 (s), 974 (m), 763 (m) cm⁻¹; **HRMS** (CI): calculated for C₁₁H₂₂NO₂ [M+H⁺] : 200.1651, found: 200.1650 (-0.5 ppm).

### Hexanoic acid [(1S,2R)-2-((E)-cyclooct-1-enyl)-2-hydroxy-1-hydroxymethyl-ethyl]-amide (3)

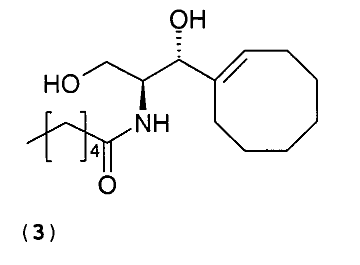

50 mg (250 µmol) (1R,2S)-2-Amino-1-((E)-cyclooct-1-enyl)-propane-1,3-diol are solved in 25 ml THF, 0.50 ml (3.61 mmol) triethylamine added and the resulting solutin cooled to -78 °C. 31.2 mg (250 *µ*mol) caproylchloride in 5 ml THF are slowly added and the solution is stirrred at -78 °C for 30 min. The reaction is terminated with 25 ml saturated NaHCO₃ solution and the mixture extracted three times with 50 ml ethylacetate. The combined organic layers are dried over MgSO₄ and evaporated. The crude product is washed with little pentane and sucked dry. 69.2 mg (232 *µ*mol, 93 %) of pure 3 are obtained.

^{**1**}**H-NMR** (360 MHz, CDCl₃, 297 K): δ [ppm] = 1.30-1.61 (m, 12H), 1.65-1.75 (m, 2H), 2.12 (dt, 2H, ³*J* = 7.6 Hz, ³*J* = 4.9 Hz), 2.19-2.43 (m, 4H), 3.47 (dd, 1H, ³*J* = 3.8 Hz, ³*J* = 11.1 Hz), 3.87 (dd, 1H, ³*J* = 3.6 Hz, ³*J* = 6.6 Hz), 3.93 (dd, 1H, ³*J* = 3.5 Hz, ²*J* = 11.1 Hz), 3.96 (d, 1H, ³*J* = 7.1 Hz), 5.68 (t, 1H, ³*J* = 8.5 Hz), 6.28 (d, 1H, ³*J* = 7.5 Hz); ^{**13**}**C-NMR** (90.56 MHz, CDCl₃, 297 K): δ [ppm] = 14.1, 25.3, 25.5, 26.6, 28.8, 30.6, 55.8, 63.9, 76.8, 126.1, 133.9, 171.0; **MS** (CI+, *i-Butane):* m/z = 298 MH⁺], 280, 239, 114, 97, 85, 71; **IR** (KBr): 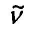 = 3392 (br), 2925 (s), 2854 (s), 1654 (s), 1567 (s), 1378 (m), 1231 (m), 1086 (s), 745 (m) cm⁻¹; **HRMS** (CI): calculated for C₁₇H₃₂NO₃ [M+H⁺] : 298.2382, found: 298.2383 (+0.3 ppm).

### 2-[((R)-2,2-Dimethyl-[1,3]dioxolan-4-yl)-hydroxy-methyl]-cyclohex-2-enone (9)

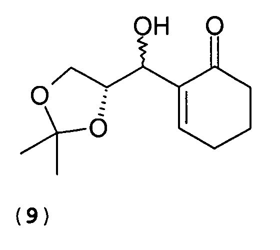

19.23 g (200 mmol) 2-Cyclohexene-1-on and 4.89 g (40 mmol) DMAP are added to a solution of 26.03 g (200 mmol) (R)-2,2-Dimethyl-[1,3]dioxolane-4-carbaldehyde in 300 ml of a mixture of water:THF = 1:1. The reaction mixture is stirred at room temperature for 96 h, diluted with 500 ml ethylacetate and the reaction is quenched by addition of 100 ml 1N hydrochloric acid. The organic layer is separated and the aqueous layer washed two times with 200 ml ethylacetate. The combined organic layers are washed with saturated NaHCO₃ solution and Brine, dried over MgSO₄ and purified by column chromatography (silica gel // ethylacetate:*n*-hexane = 2:1, R_{f} (9) - 0.61) to yield 24.0 g (106 mmol, 53 %) of the pure product (**9**).

^{**1**}**H-NMR** (360 MHz, CDCl₃, 297 K): δ [ppm] = 1.34$~(s, 3H), 1.40 (s, 3H), 1.96-2.03 (m, 2H), 2.33-2.51 (m, 4H), 3.84-3.92 (m, 1H), 4.21-4.36 (m, 1H), 4.78 (dd, 2H, ³*J* = 4.9 Hz, ⁴*J* = 1.1 Hz), 7.11 (td, 2H, ³*J* = 3.9 Hz, ⁴*J* = 0.9 Hz) ; ^{**13**}**C-NMR** (90.56 MHz, CDCl₃, 297 K): δ [ppm] = 22.7, 25.9, 25.7, 26.5, 38.5, 64.8, 68.7, 78.4, 109.2, 135.6, 149.4, 195.2; **MS** (CI+, *i*-Butane) : m/z = 227 [MH⁺], 209, 179, 169, 131, 123, 97; **IR** (Film): 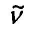 = 3435 (br), 2959 (s), 2931 (s), 2872 (w), 1665 (s), 1651 s), 1376 (m), 1243 (s), 1209 (m), 1062 (s), 890 (s) cm⁻¹; **HRMS** (CI): calculated for C₁₂H₁₉O₄ [M+H⁺]: 227.1283, found: 227.1286 (+1.3 ppm).

### 2-[((R)-2,2-Dimethyl-(1,3]dioxolan-4-yl)-trimethylsilanyloxymethyl]-cyclohex-2-enone (10)

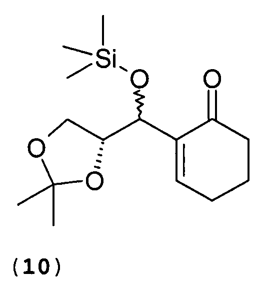

A solution of 12.0 g (53.0 mmol) **9** in 300 ml THF is cooled to 0 °C. At this temperature a solution of 5.98 g (55 mmol) trimethylsilylchloride in 40 ml THF is added followed by 10.1 g (100 mmol) triethylamine. The reaction mixture is kept for 7.5 h at reflux. After cooling to room temperature the mixture is given to 1.5 1 diethylether and 500 ml of a 1M NaH₂PO₄ solution. The organic layer is separated and the aqueous layer extracted two times with 500 ml diethylether. The combined organic layers are washed with saturated NaHCO₃ solution, dried over MgSO₄ and evaporated. The following chromatographic workup (silica gel // ethylacetate:n-hexane = 2:1, R_{f}(**10**) =0.63) yields 14.7 g (49.3 mmol, 93 %) pure 10.

^{**1**}**H-NMR** (360 MHz, CDCl₃, 297 K): δ [ppm] = 0.08$~(s, 9H), 1.33 (s, 3H), 1.39 (s, 3H), 1.95-2.02 (m, 2H), 2.35-2.50 (m, 4H), 3.80-3.89 (m, 1H), 4.05-4.18 (m, 1H), 4.72 (dd, 2H, ³*J* = 4.8 Hz, ⁴*J* = 1.1 Hz), 7.12 (td, 1H, ³*J* = 3.9 Hz, ⁴*J* = 0.8 Hz); ^{**13**}**C-NMR** (90.56 MHz, CDCl₃ 297 K): δ [ppm] = 0.2, 22.6, 25.8, 25.5, 26.3, 38.4, 65.1, 67.4, 78.5, 109.3, 139.3, 147.7, 198.0; **MS** (CI+, *i-Butane):* m/z = 299 ~ [MH⁺], 241, 225, 223, 183, 151, 101; **IR** (film): 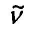 = 2985 (m), 2955 (s), 2893 (m), 1673 (s), 1380 (m), 1370 (m), 1250 (s), 1213 (m), 1158 (m), 1064 (s), 1002 (w), 891 (s), 843 (s), 756 (w) cm⁻¹; **HRMS** (CI): calculated for C₁₅H₂₇O₄Si [M+H⁺] : 299.1679, found: 299.1675 (-1.3 ppm).

### 2-[((R)-2,2-Dimethyl-[1,3]dioxolan-4-yl)-trimethylsilanyloxymethyl]-cyclohex-2-enol (11)

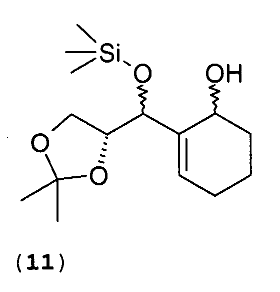

A Solution of 12.0 g (40.2 mmol) 10 in 500 ml THF is slowly added to a suspension of 5.00 g (131 mmol) LiA1H₄ in 200 ml of THF at 0 °C. The reaction mixture is allowed to warm to room temperature and stirred for 2 h. The mixture is diluted with 500 ml THF and the execessive LiAlH₄ destroyed by careful addition of 100 ml water. The same amount of 20 % NaOH is added followed by three times as much water. The precipitate is removed by filtration an washed three times with 100 diethylether. The combined organic layers are washed first with a saturated solution of NaHCO₃ and then with brine and dried over MgSO₄. Filtration and evaporation of the solvent gives the crude product which is purified by column chromatography (silica gel // ethylacetate:n-hexane = 2:1, R_{f}(**11**) = 0.52) yielding 11.4 g (37.8 mmol, 94 %) pure 11.

^{**1**}**H-NMR** (360 MHz, CDCl₃, 297 K): δ [ppm] = 0.09 (s, 9H), 1.32 (s, 3H), 1.38 (s, 3H), 1.68-1.89 (m, 4H), 2.23-2.41 (m, 2H), 3.56-3.67 (m, 1H), 4.17-4.23 (m, 1H), 4.26-4.38 (m, 1H), 4.82 (dd, 2H, ³*J* = 4.7 Hz, ⁴*J* = 1.3 Hz), 6.11-6.19 (m, 1H); ^{**13**}**C-NMR** (90.56 MHz, CDCl₃, 297 K): δ [ppm] = 0.4, 18.6, 25.3, 25.8, 25.4, 26.2, 66.0, 69.5, 73.3, 79.5, 108.1, 121.3, 141.3; **MS** (CI+, *i*-Butane): m/z = 301 [MH⁺], 283, 225, 185, 151, 101; **IR** (film): 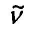 = 3480 (br), 2981 (m), 2958 (s), 2890 (m), 1376 (m), 1254 (s), 1165 (m), 1052 (s), 890 (s) cm⁻¹; **HRMS** (CI): calculated for C₁₅H₂₉O₄Si [M+H⁺] : 301.1835, found: 301.1831 (-1.3 ppm).

### Toluene-4-sulfonic acid 2-[((R)-2,2-dimethyl-[1,3]dioxolan-4-yl)-trimethylsilanyloxy-methyl]-cyclohex-2-enyl ester (12)

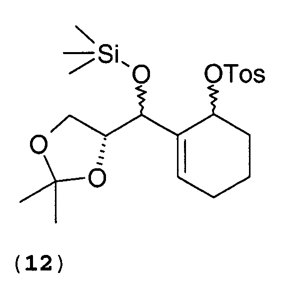

6.00 g (20.0-mmol) of the alcohol 11 are solved in 20 ml of dry chloroform and cooled to 0 °C with an ice bath. 3.62 ml (40.0 mmol) pyridine are added and then slowly 5.70 g (30 mmol) tosylchloride in small portions. The reaction is finished after 2.5 h. 60 ml of diethylether and 15 ml of water are added and the organic layer is washed with 2N HCl, a 5 % NaHCO₃ solution and water. The organic layer is dried over MgSO₄, evaporated and the residue purified by chromatographic workup (silica gel // ethylacetate:n-hexan = 1:1, R_{f}(**12**) = 0.54). Yield (12): 8.91 g(19.6 mmol, 98 %).

^{**1**}**H-NMR** (360 MHz, CDCl₃ 297 K): δ [ppm] = 0.08 (s, 9H), 1.34 (s, 3H), 1.39 (s, 3H), 1.72-2.04 (m, 4H), 2.31-2.45 (m, 2H), 3.51-3.62 (m, 1H), 4.34-4.57 (m, 2H), 4.79 (dd, 2H, ³*J* = 4.7 Hz, ⁴*J* = 1.3 Hz), 6.15-6.29 (m, 1H), 7.58 (dd, 2H, ³*J* = 7.9 Hz, ⁴*J* = 2.1 Hz), 7.74 (dd, 2H, ³*J* = 7.9 Hz, ⁴*J* = 2.1 Hz);^{**13**}**C-NMR** (90.56 MHz, CDCl₃, 297 K): δ [ppm] = 0.6, 20.9, 21.7, 25.8, 25.7, 26.5, 32.3, 66.8, 73.5, 79.4, 81.2, 107.8, 122.9, 131.2, 134.6, 138.0, 145.1; **MS** (CI+, *i-Butane):* m/z = 455 [MH⁺], 283, 185, 151, 101; **IR** (film): 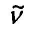 = 2976 (m), 2882 (m), 2796 (s), 1376 (m), 1234 (s), 1178 (m), 1125 (m), 1052 (s), 1032 s), 1003 (w), 890 (s) cm⁻¹; **HRMS** (CI): calculated for C₂₂H₃₅O₆Si [M+H⁺] : 455.1924, found: 455.1925 (+0.2 ppm).

### 2-[((R)-2,2-Dimethyl-[1,3]dioxolan-4-yl)-trimethylsilanyloxymethyl]-cyclohex-2-ene-carbonitrile (13)

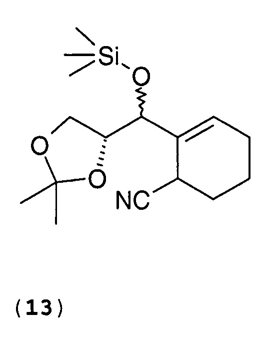

A solution of 364 mg (5.50 mmol) KCN und 529 mg (2.00 mmol) 18-Crown-6 (1,4,7,10,13,16-Hexaoxacyclooctadecane) in 100 ml acetone is added to a solution of 2.27 g (5.00 mmol) of tosylate **12** in 50 ml acetone. The mixture is heated to reflux and stirred for 36 h. After cooling to room temperature the solven is evaporated and the residue solved in a mixture of 100 ml CH₂Cl₂ and 100 ml water. The layers are separated and the aqueous layer is washed three times with 100 ml CH₂Cl₂. The combined organic layers are dried over MgSO₄ and the solvent is evaporated. The residue is purified by column chromatography (silica gel // ethylacetate:*n*-hexane = 3:2, R_{f}(**13**) = 0.52) yielding 588 mg (1.90 mmol, 38 %) pure product (**13**).

^{**1**}**H-NMR** (360 MHz, CDCl₃, 297 K): δ [ppm] = 0.07 (s, 9H), 1.34 (s, 3H), 1.39 (s, 3H), 1.45-1.96 (m, 4H), 2.17-2.34 (m, 2H), 3.82-3.91 (m, 1H), 4.12-4.19 (m, 1H), 4.43 (dd, 2H, ³*J* = 4.9 Hz, ⁴*J* = 1.3 Hz), 4.52-4.56 (m, 1H), 6.34 (m, 1H); ^{**13**}**C-NMR** (90.56 MHz, CDCl₃, 297 K): δ [ppm] = 0.5, 18.3, 23.4, 25.8, 26.6, 28.9, 30.4, 66.7, 71.3, 76.2, 109.4, 122.3, 128.5, 137.6; **MS** (CI+, *i*-Butane): m/z = 310 [MH⁺], 283, 225, 183, 151, 101; **IR** (film): 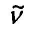 = 2976 (m), 2885 (m), 2224 (w), 1375 (m), 1245 (s), 1210 (m), 1064 (s), 890 (s), 835 (s) cm⁻¹; **HRMS** (CI): calculated for C₁₆H₂₈NO₃Si [M+H⁺] : 310.1838, found: 310.1844 (+1.9 ppm).

### C-{2-[((R)-2,2-Dimethyl-[1,3]dioxolan-4-yl)-trimethylsilanyloxymethyl]-cyclohex-2-enyl}-methylamine (14)

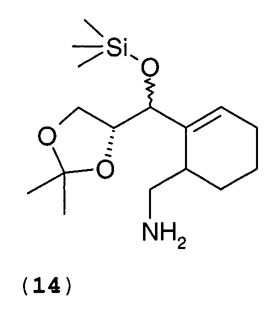

A solution of 1.55 g (5.01 mmol) **13** in 50 ml dry diethylether is slowly added to a solution of 1.00 g (26.4 mmol) LiA1H₄ in 50 ml diethylether. The reaction mixture is stirred for 1 h at reflux, cooled to room temperature and the reaction carefully quenched by addition of 10 ml of water. 10 ml of a 20 % NaOH and 30 ml water are added, the precipitate filtered off and and washed twice with 50 ml diethylether. The combined organic layers are washed with saturated NaHCO₃ solution and brine, dried over MgSO₄ and evaporated. The crude product is purified by column chromatography (silica gel // ethylacetate:n-hexan = 3:2, **R**_{**f**}**(14)** = 0.52). Yield (**14**): 1.40 g (44.6 mmol, 89 %).

^{**1**}**H-NMR** (360 MHz, CDCl₃, 297 K): δ [ppm] = 0.07 (s, 9H), 1.33 (s, 3H), 1.38 (s, 3H), 1.41-1.60 (m, 2H), 1.85-2.15 (m, 3H), 2.24-2.38 (m, 2H), 3.35 (q, 2H, ³J = 5.5 Hz), 3.58-3.65 (m, 1H), 4.21-4.31 (m, 1H), 4.35 (dd, 2H, ^{*3*}*J =* 4.8 Hz, ^{*4*}*J =* 1.1 Hz), 5.62 (m, 1H); ^{**13**}**C-NMR** (90.56 MHz, CDCl₃, 297 K): δ [ppm] = 0.6, 18.9, 22.1, 25.8, 26.6, 29.8, 36.3, 45.3, 66.6, 72.8, 79.9, 109.3, 123.2, 145.2; **MS** (CI+, *i*-Butane): m/z = 314 [MH⁺] , 287, 229, 151, 101; **IR** (Film): 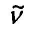 = 3360 (br), 2975 (m), 2890 (m), 1384 (m), 1230 (s), 1065 (s), 894 (s), 833 (s) cm⁻¹; **HRMS** (CI): calculated for C₂₂H₃₂NO₃Si [M+H⁺] : 314.2151, found: 314.2152 (+0.3 ppm).

### (R)-1-(6-Aminomethyl-cyclohex-1-enyl)-propane-1,2,3-triol (15)

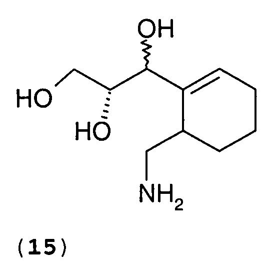

600 mg (1.91 mmol) of compound **14** are added to a mixture of 15 ml acetic acid and 5 ml water. The mixture is heated to 35 °C for 1 h, diluted with 80 ml water and extracted five times with 150 ml CH₂Cl₂. The combined organic layers are washed with a saturated NaHCO₃ solution, dried over MgSO₄ and the solvent evaporated. The resulting yellowish solid is purified by multiple recristallization from methanol/diethylether, yielding 269 mg (1.34 mmol, 70 %) of pure colorless **15**.

^{**1**}**H-NMR** (360 MHz, CDCl₃, 297 K): δ [ppm] = 1.60-1.74 (m, 2H), 1.84-2.19 (m, 3H), 2.25-2.39 (m, 2H), 3.29 (q, 2H, ³*J* = 5.7 Hz), 3.75 (d, 2H, ³*J* = 5.3 Hz), 3.87-4.05 (m, 1H), 4.21-4.31 (m, 1H), 5.64 (m, 1H) ; ^{**13**}**C -NMR** (90.56 MHz, CDCl₃ 297 K): δ [ppm] = 18.3, 23.6, 29.2, 40.9, 45.6, 63.8, 74.6, 75.2, 124.5, 138.8; **MS** (CI+, *i*-Butane.): m/z = 202 [MH⁺], 184, 152, 100; **IR** (film): 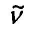 = 3370 (br), 3250 (br), 2976 (m), 2922 (s), 2857 (m), 1586 (s), 1246 (s), 1084 (s), 989 (m), 723 (m) cm⁻¹; **HRMS** (CI): calculated for C₁₀H₂₀NO₃ [M+H⁺] : 202.1443, found: 202.1443 (0.0 ppm) .

### (S)-3-Hydroxymethyl-1,2,3,4,6,7,8,8a-octahydro-isoquinolin-4-ol (8)

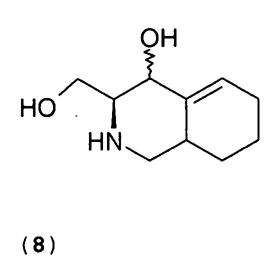

A solution of 130 mg (646 *µ*mol) substance **15** in 5.0 ml pyridine und 10 ml toluene is cooled to 0 °C and 953 mg (5.00 mmol) tosylchloride are added. The reaction mixture is stirred for 4 d at room temperature and diluted with diethylether. Successive washing first with 50 ml 1N HCl and then with 50 ml saturated NaHCO₃ solution followed by hydrolysis of remaining toluensulfonic acid ester with a solution of 4.00 g (100 mmol) NaOH in 50 ml water for 2 h at 0°°C gives the final product **8**. The organic layer is separated and the aqueous layer washed with CH₂Cl₂ several times. The combined organic layers are dried over MgSO₄ and evaporated. The residue was purified by column chromatography (silica gel // Ethylacetate:*n*-Hexan = 1:1, R_{f}(**8**) = 0.51) yielding to 51 mg (278 *µ*mol, 43 %) of pure **8**.

^{**1**}**H-NMR** (360 MHz, CDCl₃ 297 K): δ [ppm] = 1.61-1.75 (m, 3H), 1.84-2.19 (m, 2H), 2.20-2.31 (m, 2H), 3.09-3.32 (m, 3H), 3.38 (d, 2H, ³*J* = 6.2 Hz), 4.07-4.21 (m, 1H), 5.38 (m, 1H); ^{**13**}**C-NMR** (90.56 MHz, CDCl₃, 297 K): δ [ppm] = 18.3, 24.8, 28.9, 40.3, 48.1, 61.2, 63.6, 74.2, 120.1, 143.9; **MS** (CI+, *i*-Butane): m/z = 184 [MH⁺], 166, 150, 100; **IR** (KBr): 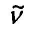 = 3364 (br), 3258 (br), 2972 (s), 2882 (s), 1580 (s), 1242 (s), 1063 (s), 1062 (m), 974 (m), 892 (s) cm⁻¹; **HRMS** (CI): calculated for C₁₀H₁₈NO₂ [M+H⁺] : 184.1338, found: 184.1336 (-1.1 ppm).

### tert-Butyl(4R,5S,2'E)-N-[2,2-Dimethyl-4-(2'-pentadecenyl)-1,3-dioxan-5-yl]carbamate (16)

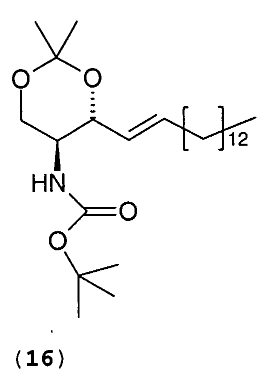

((1S,2R,3E)-2-Hydroxy-1-hydroxymethyl-heptadec-3-enyl)-carbaminsaure-tert-butylester, 100 mg (0.25 mmol) and 0.7 ml (5.71 mmol) 2,2-dimethoxypropane are combined in 2 ml dichloromethane and 62.6 mg (0.25 mmol) pyridinium-4-toluolsulfonate added. The mixture is stirred at RT over night, the solvent removed in vacuo and the residue purified by silica column chromatography (n-hexane: ethylacetate = 3: 1).
^{**1**}**H-NMR** (360 MHz, CDCl₃, 297 K): δ [ppm] = 0.88 (t, 3H, ³*J* = 6.8 Hz, CH₂CH₃), 1.25 (br, 20H), 1.42 (s, 9H), 1.48 (s, 6H), 2.04 (qt, 2H, ³*J* = 7.0 Hz, ⁴*J* = 1.3 Hz), 3.49 (br, 1H), 3.59-3.67 (m, 1H), 4.96 (d, 1H, ³*J* = 5.4 Hz), 4.99 (d, 1H, ³*J* = 5.4 Hz), 4.25-4.31(m, 1H), 5.43 (ddt, 1H, ³*J* = 7.4 Hz, ³*J* = 15.4 Hz, ⁴*J* = 1.0 Hz), 5.80 (dt, 1H, ³*J* = 6.5 Hz, ³*J* = 15.5 Hz); ^{**13**}**C-NMR** (90.56 MHz, CDCl₃, 297 K): δ [ppm] = 14.1, 19.6, 22.7, 28.3, 28.9, 29.3, 29.4, 29.5, 29.6, 29.7, 31.9, 32.4, 49.0, 63.2, 74.7, 98.7, 127.2, 136.5, 155.1; MS (CI+, *i-Butan):* m/z = 440 [MH⁺], 398, 384, 366, 326, 308, 282, 200, 144, 123, 100, 69.

### (2S,3R)-2-Amino-octadec-4-in-1,3-diol (17)

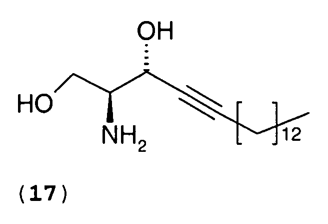

5 ml 1N HCl are added to a solution of 400 mg (1.01 mmol) tert-Butyl-(1S,2R)-N-(2-hdroxy-1-(hydroxymethyl)-heptadec-3-inyl)-carbamate in 10 ml 1,4-dioxane and the resulting mixture is heated to 100 °C for 30 min. After cooling to room temperature 5 ml 2N NaOH are added. The mixture is extracted with diethylether, the combined organic layers are washed with saturated NaCl solution and dried over MgSO₄. The solvent is evaporated and the crude product recristallized form ethylacetate/n-hexan several times, yielding to 267 mg (89 %) of a colorless solid (**17**).

^{**1**}**H-NMR** (360 MHz, d₆-DMSO, 297 K) : δ [ppm]= 0.86 (t, 3H, ³*J* = 6.7 Hz), 1.19-1.49 (m, 22H), 2.01 (td, 2H, ³*J* = 7.0 Hz, ⁴*J* = 1.7 Hz), 2.62 (q-artig, 1H, ³*J* = 5.7 Hz), 3.30 (br, 4H), 3.28 - 3.49 (m, 2H), 4.15 (dt, 1H, ³*J* = 5.4 Hz, ⁵*J* = 2.0 Hz); ^{**13**}**C-NMR** (90.56 MHz, d₆-DMSO, 297 K): δ [ppm]= 14.0, 18.0, 22.1, 28.3, 28.6, 28.7, 29.0, 29.1, 31.3, 57.8, 57.3, 62.8, 63.6, 80.5, 84.8; **MS** (CI+, i-Butan): m/z = 298 [MH⁺], 281, 280, 262, 129, 120, 101, 100, 93, 60.

### (1S,2R)-Hexadecansäure-(2-hydroxy-1-hydroxymethyl-heptadec-3-inyl)-amid (12)

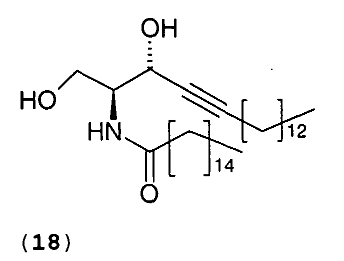

200 mg (0.67 mmol) (2S,3R)-2-Amino-octadec-4-in-1,3-diol are solved in 50 ml THF, 1 ml triethylamine is added and the resulting solution is cooled to -78 °C. At this temperature 185 mg (0.67 mmol) palmitoylchloride in 5 ml THF are slowly added and the mixture is stirred at the same temperature for 30 min. The reaction is terminated with 20 ml saturated NaHCO₃ solution and the mixture extracted three times with 30 ml ethylacetate. The combined organic layers are washed with 50 ml water, dried over MgSO₄ and the solvent is evaporated. The crude product is solved in little ethylacetate and recristallized by addition of n-pentane. The product is filtered of and washed with cold pentane yielding to 318 mg (88 %) of the pure product (**18**).

^{**1**}**H-NMR** (360 MHz, CDCl₃/CD₃OD, 297 K): δ [ppm]= 0.88 (t, 6H, ³*J* = 6.8 Hz), 1.26 (br, 22H), 1.50 (q, 2H, ³*J* = 7.6 Hz), 1.62 (q, 2H, ³*J* = 7.5 Hz), 2.31 (dt, 2H, ³*J* = 7.6 Hz, ⁵*J* = 2.1 Hz), 2.24 (t, 2H, ³*J* = 7.6 Hz), 3.61-3.72 (m, 3H), 3.85-4.00 (m, 2H), 4.51 (dt, 1H, ³*J* = 4.3 Hz, ⁵*J* = 2.0 Hz), 6.72 (d, 1H, ³*J* = 8.4 Hz); ^{**13**}**C-NNR** (90.56 MHz, CDCl₃/CD₃OD, 297 K): δ [ppm]=14.1, 18.8, 22.8, 25.9, 28.8, 29.1, 29.3, 29.4, 29.5, 29.5, 29.7, 29.8, 32.0, 36.7, 55.5, 61.8, 63.1, 77.9, 87.5, 175. 1; **MS** (CI+, *i*-Butan) : m/z = 536 [MH⁺], 534, 518, 329, 311, 298, 262, 257, 237, 111, 90, 83.

### Example 3: Apoptogenic effects of compounds 1-5

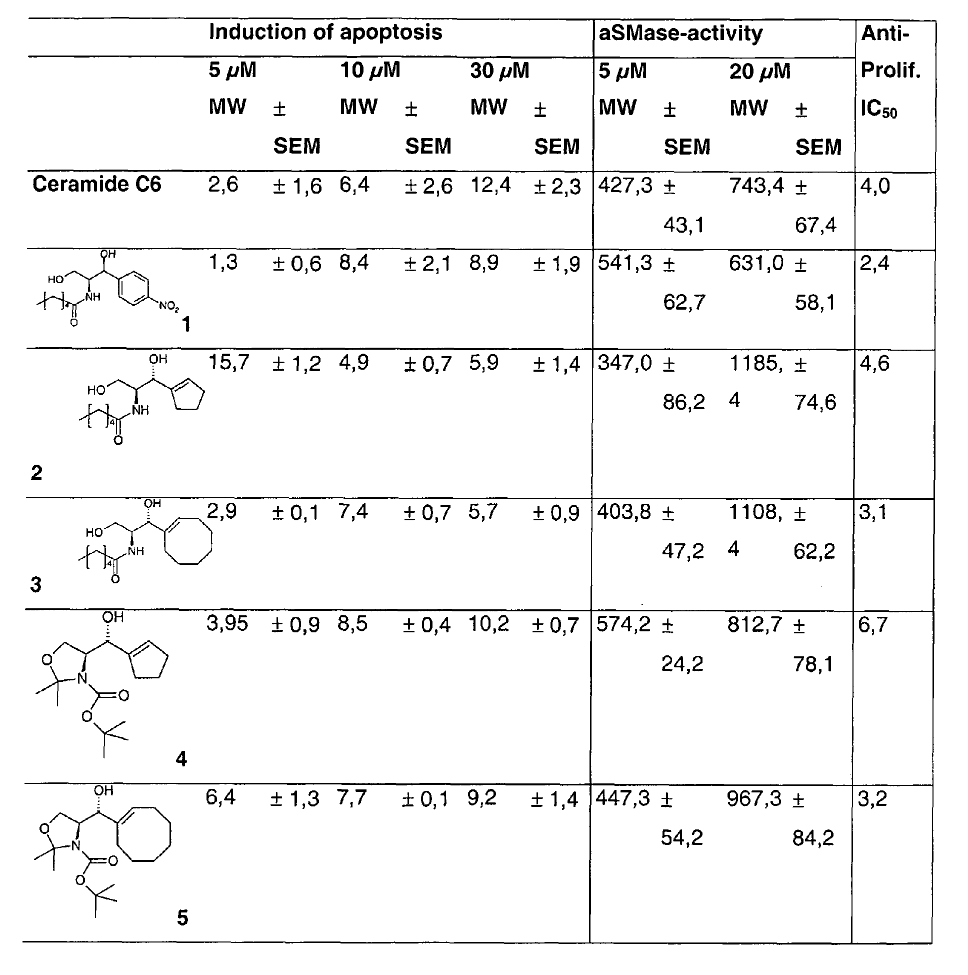

Induction of apoptosis was determined in the human adenocarcinoma cell line HT 29 as described in Example 1; spontaneous apoptosis rate was 2.48 % ± 0.58. The activation of acidic sphingomyelinase (aSMase) was analyzed in the same cell line as described in Example 1, basal activity amounts to 278,4 pmolµg⁻¹min⁻¹ ± 41,3.

Moreover, the induction of apoptosis was also elucidated by displaying the proteolytic fragmentation of an enzyme, which is considered to be specific for observing programmed cell death: compounds 1-5 features in a time and concentration dependent manner the presence of the low molecular fragment (85 kDa) of poly(ADP-ribose) polymerase, displayed by Western Blotting as described in Example 1. Next, apoptosis was investigated by COMET-assay on the basis of single cell electrophoresis after induction of apoptosis using compounds 1-8, obtaining similar results as determined by YOPRO-staining.

### Example 4: Anti-proliferative effects of compounds 1-8

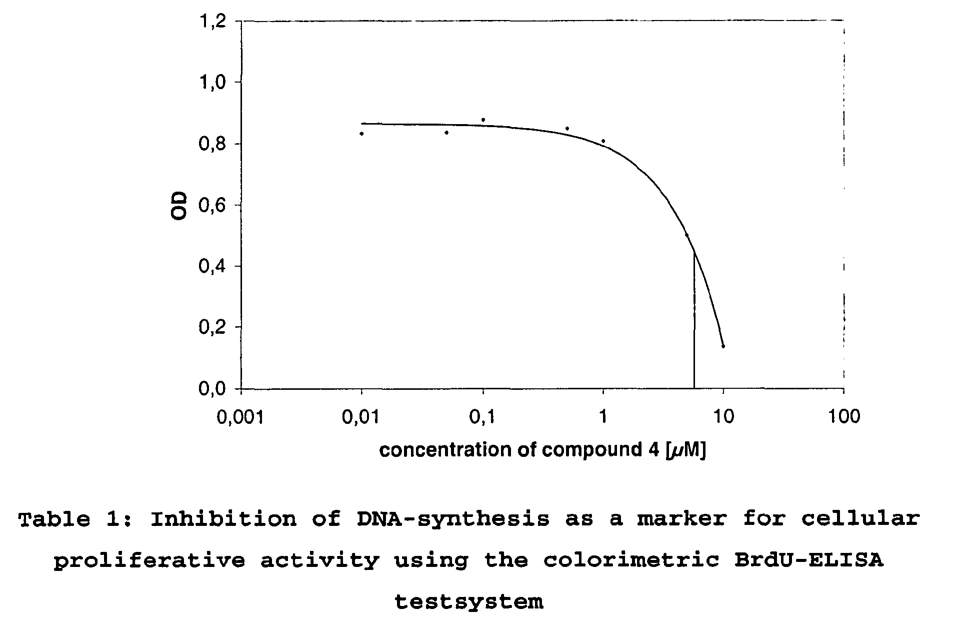

Semiconfluent HT-29 cells were incubated with compound 4 at concentration as indicated over night and DNA synthesis was analyzed on the basis of BrdU-incorporation and subsequent immunological quantification. Compound 4 inhibited cellular proliferative activity with an apparent IC50 of among 6,7 µM. For comparison, Cer shows an half maximal antiproliferative activity among 4,0 µM using similar conditions. Due to previous calculation using dependent variables over several orders of magnitudes, no standard deviation is given in Table 1 presenting the averaged half maximal concentrations for inhibition of proliferative activity.

### References

1. Kolesnick RN, Kronke M (1998) Regulation of ceramide production and apoptosis. *Annu. Rev. Physiol.,* **60,** 643-665.
2. Hannun YA, Obeid LM (1997) Mechanisms of ceramide-mediated apoptosis. *Adv. Exp. Med. Biol.* **407,** 145-149.
3. Wong ML, Xie B, Beatini N, Phu P, Marathe S, Johns A, Gold PW, Hirsch E, Williams KJ, Licinio J, Tabas I (2000) Acute systemic inflammation up-regulates secretory sphingomyelinase *in vivo:* a possible link between inflammatory cytokines and atherogenesis. *Proc. Natl. Acad. Sci. U S A.* **97,** 8681-8686.
4. Hannun, YA, Obeid LM (2002) The Ceramide-centric universe of lipid-mediated cell regulation: stress encounters of the lipid kind. J. *Biol. Chem.* 277, 25847-25850.
5. Mathias S, Pena LA, Kolesnick RN (1998) Signal transduction of stress via ceramide. *Biochem. J.* **335,** 465-480.
6. Hannun, YA (1996) Functions of ceramide in coordinating cellular responses to stress. *Science* **274,** 1855-1859.
7. Di Marzio L, Moretti S, D'Alo S, Zazzeroni F, Marcellini S, Smacchia C, Alesse E, Cifone MG, De Simone C (1999) Acetyl-L-carnitine administration increases insulin-like growth factor 1 levels in asymptomatic HIV-1-infected subjects: correlation with its suppressive effect on lymphocyte apoptosis and ceramide generation. *Clin. Immunol.* **92,** 103-110.
8. Obrig TG, Seaner RM, Bentz M, Lingwood CA, Boyd B, Smith A, Narrow W (2003) Induction by sphingomyelinase of shiga toxin receptor and shiga toxin 2 sensitivity in human microvascular endothelial cells. *Infect. Immun.* **71,** 845-849.
9. Cutler RG, Mattson MP (2001) Sphingomyelin and ceramide as regulators of development and lifespan. Mech. *Ageing Dev.* **122,** 895-908.
10. Ohanian J, Ohanian V (2001) Sphingolipids in mammalian cell signalling. *Cell. Mol. Life Sci.* **58,** 2053-2068.
11. Flores I, Jones DR, Merida I (2000) Changes in the balance between mitogenic and antimitogenic lipid second messengers during proliferation, cell arrest, and apoptosis in T-lymphocytes. *FASEB J.* **14,** 1873-1875.
12. Deigner HP, Claus R, Bonaterra GA, Gehrke C, Bibak N, Blaess M, Cantz M, Metz J, Kinscherf R (2001) Ceramide induces aSMase expression: implications for oxLDL-induced apoptosis. *FASEB J.* **15,** 807-814.
13. Wolff RA, Dobrowsky RT, Bielawska A, Obeid LM, Hannun YA (1994) Role of ceramide-activated protein phosphatase in ceramide-mediated signal transduction. J. *Biol. Chem.* **269,** 19605-19609.
14. Reyes JG, Robayna IG, Delgado PS, Gonzalez IH, Aguiar JQ, Rosas FE, Fanjul LF, Galarreta CM (1996) c-Jun is a downstream target for ceramide-activated protein phosphatase in A431 cells. *J. Biol. Chem.* **271,** 21375-21380.
15. Medvedev AE, Blanco JC, Qureshi N, Vogel SN. (1999) Limited role of ceramide in lipopolysaccharide-mediated mitogen-activated protein kinase activation, transcription factor induction, and cytokine release. *J. Biol. Chem.* **274,** 9342-9350.
16. Cifone MG, De Maria R, Roncaioli P, Rippo MR, Azuma M, Lanier LL, Santoni A, Testi R (1994) Apoptotic signaling through CD95 (Fas/Apo-1) activates an acidic sphingomyelinase. *J. Exp. Med.* **180,** 1547-52.
17. Schutze, S., Potthoff, K., Machleidt, T., Berkovic, D., Wiegmann, K., and Kronke, M. (1992) TNF activates NF-kappa B by phosphatidylcholine-specific phospholipase C-induced "acidic" sphingomyelin breakdown. *Cell* **71,** 765-76.
18. Simarro, M., Calvo, J., Vila, J.M., Places, L., Padilla, O., Alberola-Ila, J., Vives, J., and Lozano, F. (1999) Signaling through CD5 involves acidic sphingomyelinase, protein kinase C-zeta, mitogen-activated protein kinase kinase, and c-Jun NH2-terminal kinase. J. *Immunol.* **162,** 5149-55.
19. Boucher, L.M., Wiegmann, K., Futterer, A., Pfeffer, K., Machleidt, T., Schütze, S., Mak, T.W., and Krönke, M. (1995) CD28 signals through acidic sphingomyelinase. *J. Exp. Med.* **181,** 2059-68.
20. Lozano, J., Menendez, S., Morales, A., Ehleiter, D., Liao, W.C., Wagman, R., Haimovitz-Friedman, A., Fuks, Z., and Kolesnick, R. (2001) Cell autonomous apoptosis defects in acid sphingomyelinase knockout fibroblasts. *J. Biol. Chem.* **276,** 442-448.
21. Mansat-de Mas, V., Bezombes, C., Quillet-Mary, A., Bettaieb, A., D'orgeix, A. D., Laurent, G., and Jaffrezou, J. P. (1999) Implication of radical oxygen species in ceramide generation, c-Jun N-terminal kinase activation and apoptosis induced by daunorubicin. *Mol. Pharmacol.* **56,** 867-74.
22. Claus, R., Russwurm, S., Meisner, M., Kinscherf, R., and Deigner, H.P. (2000) Modulation of the ceramide level, a novel therapeutic concept? *Curr Drug Targets* **1,** 185-205.
23. Bose, R., Verheij, M., Haimovitz-Friedman, A., Scotto, K., Fuks, Z., and Kolesnick, R. (1995) Ceramide synthase mediates daunorubicin-induced apoptosis: an alternative mechanism for generating death signals. *Cell* **82,** 405-414.
24. Andrieu-Abadie, N., Jaffrezou, J.P., Hatem, S., Laurent, G., Levade, T., and Mercaider, J. J. (1999) L-carnitine prevents doxorubicin-induced apoptosis of cardiac myocytes: role of inhibition of ceramide generation. *FASEB J.* **13,** 1501-1510.
25. Ravid T, Tsaba A, Gee P, Rasooly R, Medina EA, Goldkorn T (2003) Ceramide accumulation precedes caspase-3 activation during apoptosis of A549 human lung adenocarcinoma cells. Am. J. *Physiol. Lung Cell. Mol. Physiol.* **284,** L1082-1092.
26. Goggel R, Winoto-Morbach S, Vielhaber G, Imai Y, Lindner K, Brade L, Brade H, Ehlers S, Slutsky AS, Schutze S, Gulbins E, Uhlig S. PAF-mediated pulmonary edema: a new role for acid sphingomyelinase and ceramide. *Nat. Med.* 2004 Jan 4 [Epub ahead of print].
27. Zhang DX, Zou AP, Li PL (2003) Ceramide-induced activation of NADPH oxidase and endothelial dysfunction in small coronary arteries. Am. *J. Physiol. Heart Circ. Physiol.* **284,** H605-612.
28. Idziorek T, Estaquier J, De Bels F, Ameisen JC (1995) YOPRO-1 permits cytofluorometric analysis of programmed cell death (apoptosis) without interfering with cell viability. *J. Immunol. Methods.* 185, 249-258.
29. Whitacre CM, Berger NA (1997) Factors affecting topotecan-induced programmed cell death: adhesion protects cells from apoptosis and impairs cleavage of poly(ADP-ribose)polymerase. *Cancer Res.* **57,** 2157-2163.
30. O'Callaghan YC, Woods JA, O'Brien NM (2001) Limitations of the single-cell gel electrophoresis assay to monitor apoptosis in U937 and HepG2 cells exposed to 7beta-hydroxycholesterol. *Biochem. Pharmacol.* **61**, 1217-1226.
31. Loidl A, Claus R, Deigner HP, Hermetter A (2002) High-precision fluorescence assay for sphingomyelinase activity of isolated enzymes and cell lysates. *J. Lipid Res.* **43,** 815-823.

## Claims

1. A compound of the general formula A or B: 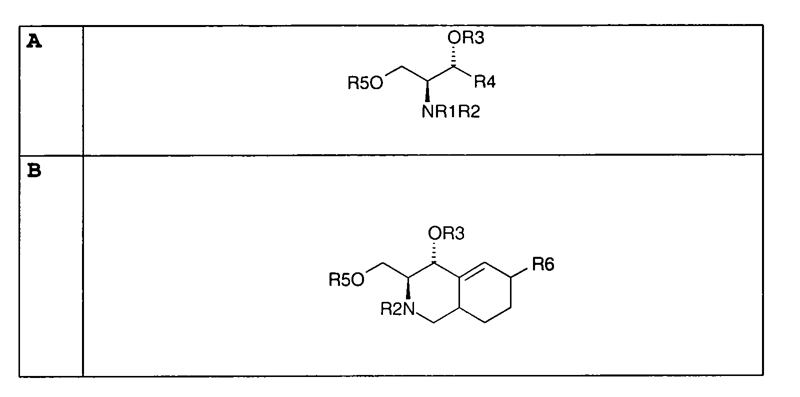 wherein
the residues R1, R2 and R3 which can be equal or different are H, CO-alkyl, CO-alkenyl, CO- aralkyl, CO-phenyl, BOC, COCF₂-alkyl, COCF₂-aralkyl, COCF₂-phenyl, a C₁-C₂₀ alkyl, alkenyl, alkinyl, aralkyl, aralkenyl residue, phenyl or substituted phenyl, a C₃-C₁₀ cycloalkyl, cycloalkenyl or substituted cycloalkenyl;
the residues R4 and R6 which can be equal or different are CO-alkyl, CO-alkenyl, CO-aralkyl, CO-phenyl, BOC, COCF₂-alkyl, COCF₂-aralkyl, COCF₂-phenyl, C₅-C₂₀ alkyl, alkenyl, alkinyl, aralkyl, aralkenyl residue, COOH, CONH₂, CONH-alkyl, CON(alkyl)₂ residue wherein alkyl - C₁-C₁₀, a nitro, an amino, alkylamino residue wherein alkyl - C₁-C₂₀, an aryl, aralkyl, alkoxyaryl residue, a halogen atom, phenyl or substituted phenyl, a C₃-C₁₀ cycloalkyl, cycloalkenyl or substituted cycloalkenyl, a C₁-C₂₀ O, S-Alkyl, alkenyl, -alkinyl, -aralkyl, -aralkenyl ether, -phenyl or substituted phenyl ether, a C₃-C₈ O, S-cycloalkenyl or substituted cycloalkenylether, -epoxyalkyl, -aryl, alkenyl, or aziridinoalkyl, -aryl, -alkenyl; and
the residue R5 is an substituted or unsubstituted alkyl, alkenyl, alkinyl, aralkyl, aralkenyl phosphate ester, phosphonic- or phosphinic acid ester, a sulfonic- or sulfinic-acid ester or a biologically active isostere thereof;
or a pharmaceutically acceptable salt thereof.

2. A compound according to claim 1, which is
(a) Hexanoic acid [(1S,2S)-2-hydroxy-1-hydroxy-methyl-2-(4-nitro-phenyl)-ethyl]-amide 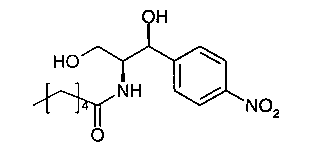
(b) Hexanoic acid (2-cyclo-pent-1-enyl-2-hydroxy-1-hydroxymethyl-ethyl)-amide 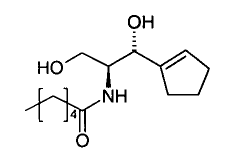
(c) Hexanoic acid [(1S,2R)-2-cyclooct-1-enyl-2-hydroxy-1-hydroxy-methyl-ethyl]-amid 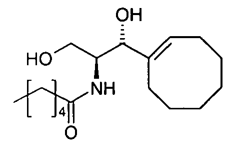
(d) 4-(Cyclopent-1-enyl-hydroxy-methyl)-2,2-dimethyloxazolidine-3-carboxylic acid tert-butyl ester 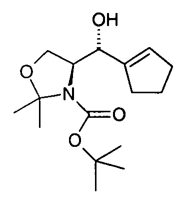
(e) 4-(Cyclooct-1-enyl-hydroxy-methyl)-2,2-dimethyl oxazolidine-3-carboxylic acid tert-butyl ester 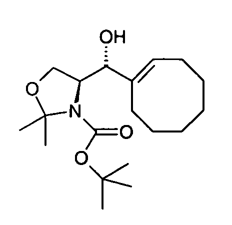
(f) (1S,8R)-5,5-Dimethyl-1-pentadec-1-enyl-dihydrooxazolo[3,4-c]-oxazol-3-one 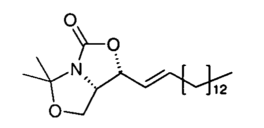
(g) (5S)-5[(1R)-1-Hydroxy-hexadec-2-enyl]-morpholin-3-one 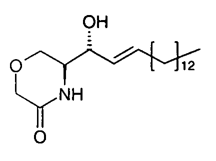 or
(h) 3-Hydroxymethyl-1,2,3,4,6,7,8,8a-octa-hydro-isoquinolin-4-ol

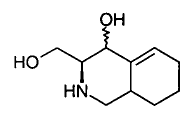

3. A pharmaceutical composition comprising a pharmaceutically effective amount of a compound according to claim 1 or 2 in combination with a pharmaceutically acceptable carrier.

4. Use of a compound according to claim 1 or 2 for the preparation of a pharmaceutical composition for cancer therapy and/or for improving the efficiency of a cytostatic and/or radiation therapy.

5. Use of a compound according to claim 1 or 2 for the preparation of a pharmaceutical composition for preventing or reducing the formation of resistances by a cytostatic and/or radiation therapy.

6. Use according to claim 4 or 5, wherein the cytostatic agent is an intercalating agent or antimitotic agent.

7. Use according to claim 6, wherein the intercalating agent is daunorubicin and/or the antimitotic agent is a vinca alcaloid.

8. Use according to claim 7, wherein the vinca alcaloid is vinblastine.

9. Use of a compound according to claim 1 or 2 for the preparartion of a pharmaceutical composition for inhibiting proliferation of and/or inducing apoptosis in cells.

10. Use of a compound according to claim 1 or 2 for the preparation of a pharmaceutical composition for therapy and/or prophylaxis of cancer, colorectal hyperproliferation or inhibition of increased angiogenesis.

11. Use according to claim 10, wherein the cancer therapy is an adjuvant therapy wherein said compound is combined with at least one additional anti-cancer agent of an independent or complementary mechanism.

12. Use according to claim 10 or 11, wherein the cancer is a lung adenocarcinoma.

13. Use of a compound according to claim 1 or 2 for the preparation of a pharmaceutical composition for therapy and/or prophylaxis of asthma, rheumatoide arthritis, autoimmune arthritis, morbus crohn, colitis ulcerosa, systemic inflammation, sepsis, acute respiratory distress syndrome (ARDS), chronic obstructive and/or restrictive lung disease or a disease associated with hyperproliferation and/or epithelial disorder.

14. Use according to claim 13, wherein said epithelial disorder is psoriasis, comedogenesis, chronic proliferative dermatitis, epithelializating or chronic wounds, systemic lupus erythematosus, endometriosis or chronic irritant contact dermatitis.

15. Use of a compound according to claim 1 or 2 for the preparation of a pharmaceutical composition for inhibiting proliferation of human vascular cells in vessels treated with stents impregnated with said compounds and/or containing devices for retarded or continous delivery.

16. Use of a compound according to claim 1 or 2 for the preparation of a pharmaceutical composition for inducing positive inotropic effects in ventricular myocytes or for an improvement of the left ventricular function.

17. Use of a compound according to claim 1 or 2 for the preparation of a pharmaceutical composition for (a) preventing or attenuating apoptosis of neuronal cells or (b) inhibiting or recucing the consequences of stroke and/or ischaemic insult.

18. Use of a compound according to claim 1 or 2 for the preparation of a pharmaceutical composition for preventing and/or treating a neurodegenerative disease.

19. Use according to claim 18, wherein said neurodegenerative disease is morbus alzheimer, m. parkinson, a prion disease or amyotrophic lateral sclerosis.

20. Use of a compound according to claim 1 or 2 for the preparation of a pharmaceutical composition for prophylaxis, therapeutically monitoring and adjuvant therapeutic in cancer therapy, colorectal hyperproliferation, asthma, rheumatoide arthritis, autoimmune arthritis, morbus crohn, colitis ulcerosa, systemic inflammation, sepsis, acute respiratory distress syndrome (ARDS), chronic obstructive and/or restrictive lung disease, a disease associated with hyperproliferation and/or epithelial disorder.

21. use of a compound according to claim 1 or 2 mimicking biological activities of sphingosine or sphingosine-1-phosphate for the preparation of a pharmaceutical composition for preventing or inhibiting apoptosis and antiproliferative effects due to treatment with ionizing radiation, cytostatic drugs and/or chemotherapeutics and for preventing side effects thereof.
